(19) Europäisches Patentamt
European Patent Office
Office européen des brevets

(11) Veröffentlichungsnummer: **0 332 991 B1**

(12) # EUROPÄISCHE PATENTSCHRIFT

(45) Veröffentlichungstag der Patentschrift: **10.11.93**

(51) Int. Cl.[5]: **C07D 249/14**, C07D 401/12, C07D 403/12, C07D 409/12, C07D 405/12, C07D 413/12, C07D 417/12, A01N 43/653, C07D 401/14, C07D 403/14, C07D 409/14

(21) Anmeldenummer: **89103971.1**

(22) Anmeldetag: **07.03.89**

Die Akte enthält technische Angaben, die nach dem Eingang der Anmeldung eingereicht wurden und die nicht in dieser Patentschrift enthalten sind.

(54) **Substituierte Triazole.**

(30) Priorität: **18.03.88 DE 3809053**

(43) Veröffentlichungstag der Anmeldung: **20.09.89 Patentblatt 89/38**

(45) Bekanntmachung des Hinweises auf die Patenterteilung: **10.11.93 Patentblatt 93/45**

(84) Benannte Vertragsstaaten: **BE CH DE FR GB IT LI NL**

(56) Entgegenhaltungen:
**DD-A- 242 612**
**DD-A- 242 616**
**DE-A- 1 695 377**
**GB-A- 919 458**

(73) Patentinhaber: **BAYER AG**
**D-51368 Leverkusen(DE)**

(72) Erfinder: **Findeisen, Kurt, Dr.**
**In der Follmühle 10**
**D-5068 Odenthal 2(DE)**
Erfinder: **Lindig, Markus, Dr.**
**Dahlienweg 16**
**D-4010 Hilden(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6a**
**D-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

**CHEMICAL ABSTRACTS** Band 85, Nr. 11, 15th September 1976, Seite 487, Spalte 1, Abstract Nr. 77387u, Columbus, Ohio, US; M.G. VORONKOV et al.: "Basicity and protonation center of 5(3)-substitued-3(5)-amino-1,2,4-triazole"

**Beschreibung**

Die Erfindung betrifft neue substituierte Triazole, mehrere Verfahren zu ihrer Herstellung und ihre Verwendung als Herbizide

Es ist bekannt, daß bestimmte Stickstoffheterocyclen wie beispielsweise das Imidazolidin-2-on-1-carbonsäureisobutylamid (vgl. z.B. K. H. Büchel "Pflanzenschutz und Schädlingsbekämpfung" S. 170, Thieme Verlag Stuttgart 1977) herbizide Eigenschaften besitzen. In der DD-A 242 616 wird ein Verfahren zur Herstellung von 3-Heteroaryl-1,2,4-triazol-5-thiocarbonsäuramiden und deren biocide Verwendung vorgestellt.

Die DE-A 242 612 beschreibt ein Verfahren zur Herstellung von neuen 3-Carbamoyl-1,2,4-triazol-5-thiocarbonsäureamiden,die als biocide Substanzen oder Bausteine für Folgeprodukte von Interesse sind.

In der DE-A 1 695 377 werden 1,2,4-Triazol-5-carbonsäureamide beschrieben, insbesondere unter dem Aspekt als Mittel zur Verhinderung der Lichthofbildung in der fotografischen Anwendung.

In der GB-A 919 458 werden Carbamyl und Thiocarbamyl Verbindungen der 1,2,4-Triazole beschrieben,mit Hinweis auf deren herbizide Anwendbarkeit.

Schließlich sei noch auf die Literaturstelle in Chem. Abs. 85:77387 u (1976) hingewiesen, wo 5(3)-substituierte-3(5)-amino-1,2,4-triazole in Hinsicht auf ihre Basizität und ihrer Protonierungsmöglichkeit untersucht werden.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazole der allgemeinen Formel (I),

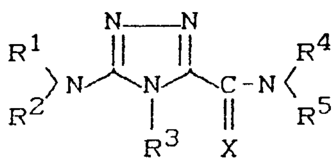

(I)

in welcher

$R^1$ und $R^2$ unabhängig voneinander jeweils für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im geradkettigen oder verzweigten Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/ oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8

Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^4$ und $R^5$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem für im Heterocyclylteil gegebenenfalls einfach oder mehrfach, gleich oder verchieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen und außerdem für jeweils gegebenenfalls einfach oder mehrfach, gleich oder verschieden substituiertes Aralkyl, Aroyl oder Aryl mit jeweils 6 bis 10 Kohlenstoffatomen im Arylteil und gegebenenfalls 1 bis 8 Kohlenstoffatomen im geradkettigen oder verzweigten Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^4$ und $R^5$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen und

X für Sauerstoff oder Schwefel steht, ausgenommen die Verbindungen, in denen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alyklteil steht.

Weiterhin wurde gefunden, daß man die neuen substituierten Triazole der allgemeinen Formel (I),

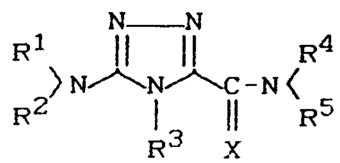

(I)

in welcher

$R^1$, $R^2$, $R^3$, $R^4$, $R^5$ und X die oben angegebenen Bedeutungen haben, ausgenommen die Verbindungen, in denen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alyklteil steht,

erhält, wenn man

(a) Aminoguanidine der Formel (II),

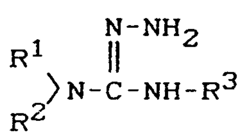

(II)

in welcher

$R^1$, $R^2$ und $R^3$ die oben angegebene Bedeutung haben,

oder deren Säureadditionssalze mit (Thio)Oxalesteramiden der Formel (III),

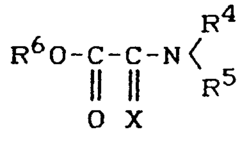

(III)

in welcher

$R^6$ für Alkyl steht und

$R^4$, $R^5$ und X die oben angegebene Bedeutung haben,

gegebenenfalls in Gegenwart eines Verdünnungsmittels und gegebenenfalls in Gegenwart eines Reaktionshilfsmittels umsetzt oder wenn man

(b) substituierte Triazolyl(thio)carbonsäureester der Formel (IV),

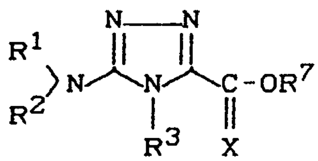 (IV)

in welcher

$R^7$ für Alkyl steht und

$R^1$, $R^2$, $R^3$ und X die oben angegebene Bedeutung haben,

mit Aminen der Formel (V),

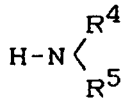 (V)

in welcher

$R^4$ und $R^5$ die oben angegebene Bedeutung haben,

gegebenenfalls in Gegenwart eines Verdünnungsmittels umsetzt.

Schließlich wurde gefunden, daß die neuen substituierten Triazole der allgemeinen Formel (I) herbizide Eigenschaften besitzen.

Überraschenderweise zeigen die erfindungsgemäßen substituierten Triazole der allgemeinen Formel (I) eine erheblich höhere herbizide Potenz gegenüber Problemunkräutern als die aus dem Stand der Technik bekannten Stickstoffheterocyclen, wie beispielsweise das Imidazolin-2-on-1-carbonsäureisobutylamid, welches eine chemisch und wirkungsmäßig naheliegende Verbindung ist.

Die erfindungsgemäßen substituierten Triazole sind durch die Formel (I) allgemein definiert. Bevorzugt sind Verbindungen der Formel (I), bei welchen

$R^1$ und $R^2$ unabhängig voneinander jeweils für Wasserstoff, Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein-bis dreifach, gleich oder verschieden substituiertes Benzyl, Phenylethyl oder Phenyl stehen, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio oder

$R^1$ und $R^2$ gemeinsam mit dem Stickstoffatom, an welches sie gebunden sind, für einen gegebenenfalls ein-bis dreifach, gleich oder verschieden substituierten Heterocyclus der Formel

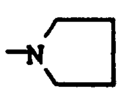 ; 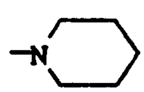 ; 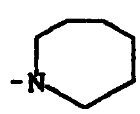 ; 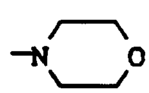 ;

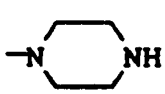 ; 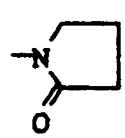 ; 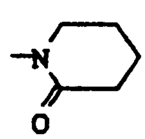 ; 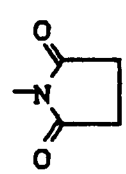 ;

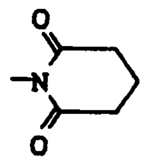 ; 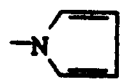 oder 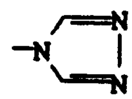 stehen,

wobei als Substituenten jeweils infrage kommen: Methyl, Ethyl, n- oder i-Propyl, Chlor oder Trifluormethyl,

$R^3$ für Methyl, Ethyl, n- oder i-Propyl, n-, i-, s- oder t-Butyl, n- oder i-Pentyl, n- oder i-Hexyl, für Allyl, Propargyl, für Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^4$ und $R^5$ unabhängig voneinander jeweils für Wasserstoff, Methyl, Ethyl, Propyl, n-, i-, s- oder t-Butyl, jeweils geradkettiges oder verzweigtes Pentyl, Hexyl, Heptyl, Octyl, Nonyl, Decyl oder Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl stehen, wobei als Substituenten jeweils infrage kommen: Fluor, Chlor, Brom, Methyl, Ethyl, n- oder i-Propyl, n-, i-, s-oder t-Butyl, Cyano, Methandiyl, Ethandiyl, Butandiyl oder Butadiendiyl;

außerdem für im Heterocyclylteil gegebenenfalls ein- bis dreifach, gleich oder verschieden substituiertes Heterocyclylmethyl, Heterocyclylpropyl oder Heterocyclylethyl stehen, wobei als Heterocyclen jeweils infrage kommen:

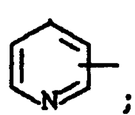 ; 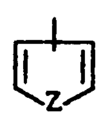 ; 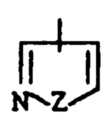 ; 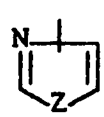 ;

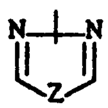 ; 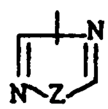 ; 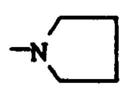 ; 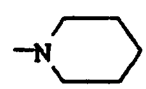 ;

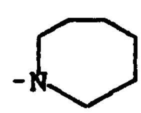 ; 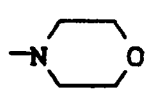 oder 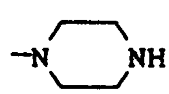 ,

wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy oder

$R^4$ und $R^5$ gemeinsam mit dem Stickstoffatom, an welches sie gebunden sind, für einen gegebenenfalls ein- bis dreifach, gleich oder verschieden substituierten Heterocyclus der Formel

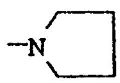 ; 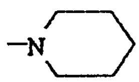 ; 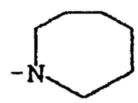 ; 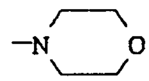 ;

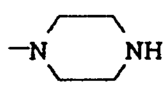 ; 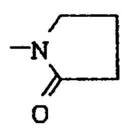 ; 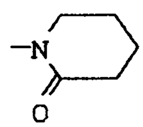 ; 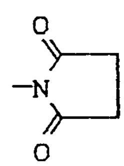 ;

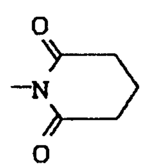 ; 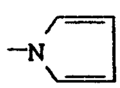 oder 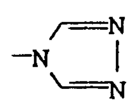 stehen,

wobei als Substituenten jeweils infrage kommen: Methyl, Ethyl, n- oder i-Propyl, Chlor oder Trifluormethyl und

X für Sauerstoff oder Schwefel steht, ausgenommen die Verbindungen, in denen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alkylteil steht.

Besonders bevorzugt sind Verbindungen der Formel (I), bei welchen

$R^1$ für Wasserstoff, Methyl oder Ethyl steht,

$R^2$ für Methyl oder Ethyl steht,

$R^3$ für Methyl oder Ethyl steht,

$R^4$ für Wasserstoff oder Methyl steht,

$R^5$ für Wasserstoff, Methyl, Ethyl, n- oder i- Propyl, n-, i-, s-oder t-Butyl, jeweils geradkettiges oder verzweigtes, insbesondere n oder iso, Pentyl, Hexyl, Heptyl, Octyl, Nonyl, Decyl oder Dodecyl, für Allyl, n- oder i-Butenyl, n- oder i-Pentenyl, n-oder i-Hexenyl, Propargyl, n- oder i-Butinyl, n-oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder

verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkylbzw. Alkenylteilen oder für jeweils gegebenenfalls ein- bis dreifach, gleich oder verschieden durch Fluor, Chlor, Brom, Methyl, Ethyl, n- oder i-Propyl, n-, i-, s- oder t-Butyl, Cyano, rtethandiyl, Ethandiyl, Butandiyl oder Butadiendiyl substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht;
außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen jeweils infrage kommen:

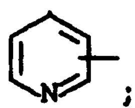 ; 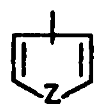 ; 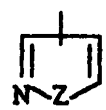 ; 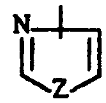 ;

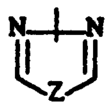 ; 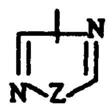 ; 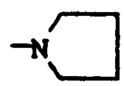 ; 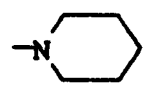 ;

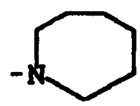 ; 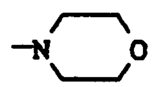 oder 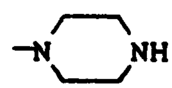 ,

wobei Z jeweils für Sauerstoff oder Schwefel steht,
außerdem für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht und

| | |
|---|---|
| X | für Sauerstoff steht, ausgenommen die Verbindungen in denen |
| $R^1$ und $R^2$ | gemeinsam mit dem Stickstoffatom, an welches sie gebunden sind, für einen gegebenenfalls substituierten Heterocyclus stehen, |
| X | für Schwefel steht, |
| $R^3$ | für Aryl mit 6 bis 10 Kohlenstoffatomen steht, |
| $R^4$ und $R^5$ | unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alyklteil steht. |

Insbesondere bevorzugt sind Verbindungen der Formel (I), bei welchen

| | |
|---|---|
| $R^1$ | für Methyl oder Ethyl steht, |
| $R^2$ | für Methyl oder Ethyl steht, |
| $R^3$ | für Methyl oder Ethyl steht, |
| $R^4$ | für Wasserstoff oder Methyl steht, |
| $R^5$ | für Wasserstoff, Methyl, Ethyl, n- oder i-Propyl, n-, i-, s- oder t-Butyl, jeweils geradkettiges oder verzweigtes, insbesondere n oder iso, Pentyl, Hexyl, Heptyl, i-Octyl, Nonyl, Decyl oder Dodecyl, für Allyl, n-oder i-Butenyl, n- oder i-Pentenyl, n-oder i-Hexenyl, Propargyl, |

n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, oder Alkoxyalkyl mit 1 bis 4 Kohlenstoffatomen in den einzelnen Alkylteilen oder für jeweils gegebenenfalls ein- bis dreifach, gleich oder verschieden durch Fluor, Chlor, Brom, Methyl, Ethyl, n- oder i-Propyl, n-, i-, s- oder t-Butyl, Cyano substituiertes Cyclopropyl, Cyclopropylmethyl, Cyclopropylethyl, Cyclobutyl Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht; außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Pyridylmethyl steht, außerdem für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl substuiertes, gegebenenfalls geradkettiges oder verzweigtes Benzyl, Phenylethyl, Phenylpropyl, Phenylbutyl, Phenylpentyl, Phenylhexyl, Phenylheptyl, steht und

X für Sauerstoff steht, ausgenommen die Verbindungen, in denen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alyklteil steht,

Im einzelnen seien außer den bei den Herstellungsbeispielen genannten Verbindungen die folgenden substituierten Triazole der allgemeinen Formel (I) genannt:

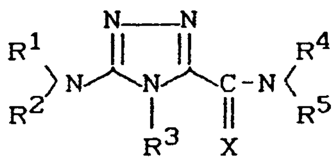

(I)

| $R^1$ | $R^2$ | $R^3$ | $R^4$ | $R^5$ | X |
|---|---|---|---|---|---|
| $CH_3$ | $CH_3$ | $C_2H_5$ | H | $-C(CH_3)_3$ | O |
| H | $CH_3$ | $CH_3$ | H | 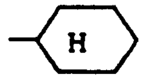 | O |
| H | $CH_3$ | $C_2H_5$ | H | $-CH_2-C(CH_3)_3$ | O |
| H | $CH_3$ | $C_2H_5$ | H | 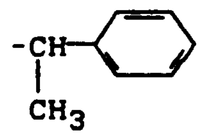 | O |
| $CH_3$ | $CH_3$ | $CH_3$ | $CH_3$ | $-C(CH_3)_3$ | O |
| $CH_3$ | $CH_3$ | $C_2H_5$ | $CH_3$ | $-C(CH_3)_3$ | O |
| $C_2H_5$ | $C_2H_5$ | $CH_3$ | H | $CH_3$<br>$-C-CF_3$<br>$CH_3$ | O |
| H | $C_2H_5$ | $CH_3$ | H | $-C(CH_3)_3$ | O |
| H | $CH_3$ | $CH_3$ | $CH_3$ | $-C(CH_3)_3$ | O |
| $CH_3$ | $CH_3$ | $CH_3$ | H | $-CH-CH=N-OCH_3$<br>$CH_3$ | O |
| $CH_3$ | $CH_3$ | $CH_3$ | $CH_3$ | $-CH-CH=N-OCH_3$<br>$CH_3$ | O |
| H | $CH_3$ | $CH_3$ | H | $-CH-CH=N-OCH_3$<br>$CH_3$ | O |

Verwendet man beispielsweise 1-Amino-2,2,3-trimethylguanidinium Hydrochlorid und Oxalsäuremonoethylester-N-allylamid als Ausgangsstoffe, so läßt sich der Reaktionsablauf des erfindungsgemäßen Verfahrens (a) durch das folgende Formelschema darstellen:

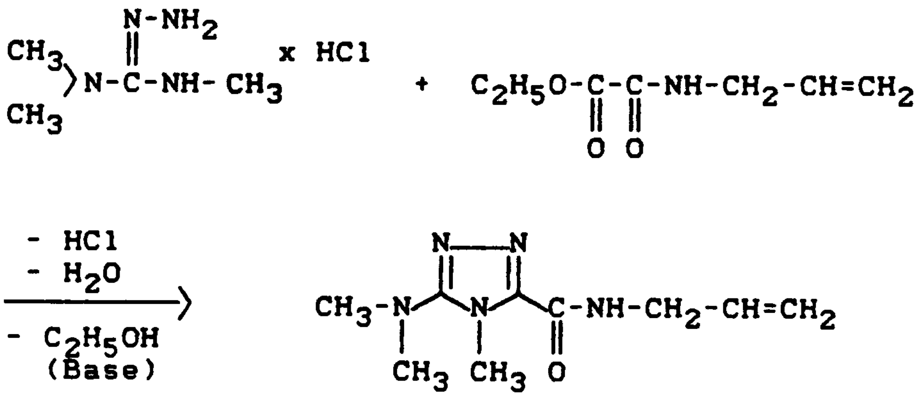


Verwendet man beispielsweise 5-Dimethylamino-4-methyl-4H-1,2,4-triazol-3-yl-carbonsäureethylester und Benzylamin als Ausgangsstoffe, so läßt sich der Reaktionsablauf des erfindungsgemäß Verfahrens (b) durch das folgende Formelschema darstellen:

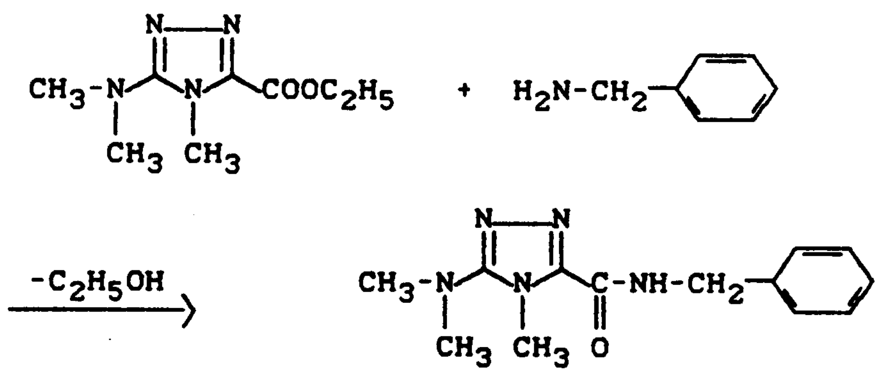


Die zur Durchführung des erfindungsgemäßen Verfahrens (a) als Ausgangsstoffe benötigten Aminoguanidine sind durch die Formel (II) allgemein definiert. In dieser Formel (II) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aminoguanidine der Formel (II) bzw. deren Säureadditionssalze, wie insbesondere deren Hydrochloride oder Hydrobromide, sind bekannt oder erhältlich in Analogie zu bekannten Verfahren, beispielsweise wenn man die allgemein bekannten Harnstoffe der Formel (VI),

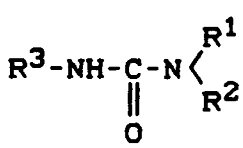 (VI)

in welcher

$R^1$, $R^2$ und $R^3$ die oben angegebene Bedeutung haben,

zunächst in einer 1. Stufe mit Phosgen gegebenenfalls in Gegenwart eines Verdünnungsmittels wie beispielsweise Toluol oder Acetonitril bei Temperaturen zwischen + 10 °C und + 150 °C umsetzt, und die so erhältlichen Formamidinhydrochloride der Formel (VII),

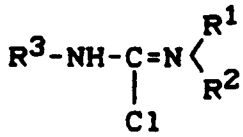 x HCl (VII)

in welcher

$R^1$, $R^2$ und $R^3$ die oben angegebene Bedeutung haben,

in einer 2. Stufe mit Hydrazinhydrat, gegebenenfalls in Gegenwart eines Verdünnungsmittels wie beispielsweise Isopropanol oder Dichlormethan bei Temperaturen zwischen - 10 °C und + 60 °C umsetzt (vgl. z.B. J. org. Chem. 19, 1807 [1954]; Bull. Soc. Chim. Fr. 1975, 1649; US 2 845 458).

Harnstoffe der Formel (VI) sind allgemein bekannte Verbindungen der organischen Chemie.

Die zur Durchführung des erfindungsgemäßen Verfahrens (a) weiterhin als Ausgangsstoffe benötigten (Thio)Oxalesteramide sind durch die Formel (III) allgemein definiert. In dieser Formel (III) stehen $R^4$, $R^5$ und X vorzugsweise für diejenigen Reste, die bereits im Zusammenhang mit der Beschreibung der erfindungsgemäßen Stoffe der Formel (I) als bevorzugt für diese Substituenten genannt wurden.

$R^6$ steht vorzugsweise für geradkettiges oder verzweigtes Alkyl mit 1 bis 4 Kohlenstoffatomen, insbesondere für Methyl oder Ethyl.

Die (Thio)Oxalesteramide der Formel (III) sind allgemein bekannt oder erhältlich in Analogie zu allgemein bekannten Verfahren (vgl. z.B. Houben-Weyl, "Methoden der organischen Chemie" Band VIII, S. 659, Thieme Verlag Stuttgart; US-PS 28 57 390; Compt. rend. Acad. Sci. 230, 848 [1950]; GB 15 78 719; DE-OS 28 19 878; DE-OS 12 27 451).

Die zur Durchführung des erfindungsgemäßen Verfahrens (b) als Ausgangsstoffe benötigten Triazolyl(thio)carbonsäureester sind durch die Formel (IV) allgemein definiert. In dieser Formel (IV) stehen $R^1$, $R^2$, $R^3$ und X vorzugsweise für diejenigen Reste, die bereits im Zusammenhang mit der Beschreibung der erfindungsgemäßen Stoffe der Formel (I) als bevorzugt für diese Substituenten genannt wurden.

$R^7$ steht vorzugsweise für geradkettiges oder verzweigtes Alkyl mit 1 bis 4 Kohlenstoffatomen, insbesondere für Methyl oder Ethyl.

Die Triazolyl(thio)carbonsäureester der Formel (IV) sind teilweise bekannt oder erhältlich in Analogie zu bekannten Verfahren (vgl. z.B. EP 48 555; EP 16 565; EP 150 507; Ref. Zh., Khim 1984, Abstr. No. 3 ZH 271 bzw. C.A. 101: 54 999e) beispielsweise wenn man Aminoguanidine der Formel (II),

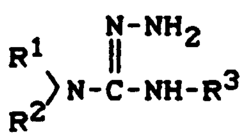 (II)

in welcher

$R^1$, $R^2$ und $R^3$ die oben angegebene Bedeutung haben,

oder deren Säureadditionssalze, wie insbesondere deren Hydrochloride, mit (Thio)Oxalsäureestern der Formel (VIII),

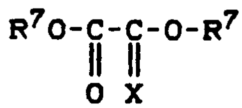 (VIII)

in welcher

$R^7$ und X die oben angegebene Bedeutung haben,

gegebenenfalls in Gegenwart eines Verdünnungsmittels, wie beispielsweise Acetonitril oder 1,2-Dichlorbenzol bei Temperaturen zwischen 70 °C und 250 °C umsetzt.

(Thio)Oxalsäureester der Formel (VIII) sind allgemein bekannt oder erhältlich in Analogie zu bekannten Verfahren (vgl. z.B. Liebigs Ann. Chem. 1974, 671-689 oder Tetrahedron 33, 259-263 [1977]).

Die zur Durchführung des erfindungsgemäßen Verfahrens (b) als Ausgangsstoffe benötigten Amine sind durch die Formel (V) allgemein definiert. In dieser Formel (V) stehen $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Alkohole, wie Methanol, Ethanol oder Propanol.

Das erfindungsgemäße Verfahren (a) wird gegebenenfalls in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 30 °C und 150 °C, vorzugsweise bei Temperaturen zwischen 50 °C und 80 °C.

Zur Durchführung des erfindungsgemäßen Verfahrens (a) setzt man pro Mol an Aminoguanidin der Formel (II) bzw. eines entsprechenden Säureadditionssalzes im allgemeinen 1.0 bis 1.5 Mol, vorzugsweise 1.0 bis 1.2 Mol an (Thio)Oxalesteramid der Formel (III) und gegebenenfalls 1.0 bis 5.0 Mol, vorzugsweise 1.0 bis 2.5 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens (b) in einem größeren Bereich variiert werden. Im allgemeinen arbeitet man bei Temperaturen zwischen 50 °C und 250 °C, vorzugsweise bei Temperaturen zwischen 100 °C und 220 °C.

Das erfindungsgemäße Verfahren (b) kann gegebenenfalls auch unter Druck durchgeführt werden. Vorzugsweise arbeitet man in Druckbereichen zwischen 1.0 und 50.0 bar, insbesondere in Druckbereichen zwischen 3.0 und 20.0 bar.

Zur Durchführung des erfindungsgemäßen Verfahren (b) setzt man pro Mol an substituiertem Triazolyl(thio)carbonsäureester der Formel (IV) im allgemeinen 1.0 bis 1.5 Mol, vorzugsweise 1.0 bis 1.2 Mol an Amin der Formel (V) ein.
Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung dikotyler Unkräuter insbesondere in monokotylen Kulturen, wie beispielsweise Weizen oder Mais einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gestein wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 2,4-Dichlorphenoxypropionsäure (2,4-DP); 4-(2,4-Dichlorphenoxy)-buttersäure (2,4-DB); (2-Methyl-4-chlorphenoxy)-essigsäure (MCPA); (4-Chlor-2-methylphenoxy)-propionsäure (MCPP); 3,5,6-Trichlor-2-pyridyloxyessigsäure (TRICLOPYR); 2-[4-(2,4-Dichlorphenoxy)-phenoxy]-propionsäure, deren Methyl- oder deren Ethylester (DICLOFOP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamno)-1,3,5-triazin (CYANAZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON) sind gegebenenfalls von Vorteil. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

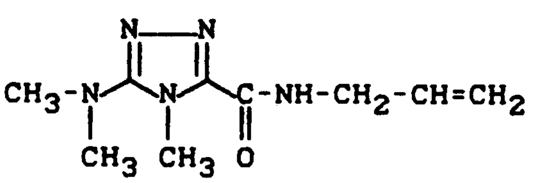

(Verfahren a)

13,73 g (0.09 Mol) 1-Amino-2,2,3-trimethylguanidinium Hydrochlorid, 14,13 g (0.09 Mol) Oxalsäuremonoethylester-N-allylamid und 9,72 g (0.18 Mol) Natriummethylat werden in 200 ml Ethanol 30 Minuten bei 80 °C gerührt, filtriert und in Vakuum eingeengt. Der Rückstand wird in 200 ml Dichlormethan aufgenommen, mit Wasser gewaschen, über Natriumsulfat getrocknet, im Vakuum eingeengt und aus Ligroin/Toluol (95:5) umkristallisiert.

Man erhält 4,07 g (22 % der Theorie) an 5-Dimethylamino-4-methyl-4H-1,2,4-triazol-3-yl-carbonsäure-N-allylamid vom Schmelzpunkt 63 °C - 65 °C.

Beispiel 2

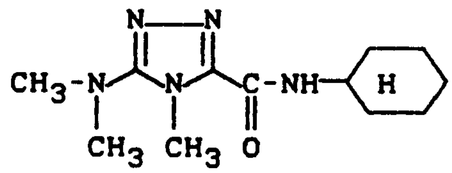

(Verfahren a)

61 g (0.4 Mol) 1-Amino-2,2,3-trimethylguanidinium Hydrochlorid, 79,6 g (0.4 Mol) Oxalsäuremonoethylester-N-cyclohexylamid und 43,2 g (0.8 Mol) Natriummethylat werden in 800 ml Ethanol 1 Stunde bei Rückflußtemperatur gerührt, auf Raumtemperatur abgekühlt, mit Essigsäure angesäuert, filtriert, das Filtrat im Vakuum eingeengt, der Rückstand in Dichlormethan aufgenommen, dreimal mit jeweils 150 ml Wasser gewaschen, über Natriumsulfat getrocknet und im Vakuum eingeengt.

Man erhält 52,6 g (53 % der Theorie) an 5-Dimethylamino-4-methyl-4H-1,2,4-triazol-3-yl-carbonsäure-N-cyclohexylamid vom Schmelzpunkt 60 °C - 62 °C.

Herstellung der Ausgangsverbindungen

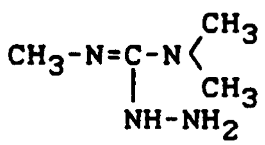 x HCl

Zu 50 g (1 Mol) Hydrazinhydrat in 300 ml Isopropanol tropft man bei 20 °C bis 25 °C unter Rühren innerhalb von 30 Minuten eine Lösung von 78,5 g (0,5 Mol) Chlortrimethylformamidiniumhydrochlorid in 250 ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

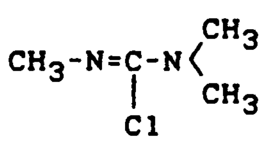 x HCl

In eine Mischung aus 510 g (5 Mol) N,N,N'-trimethylharnstoff und 3 l Chlorbenzol leitet man bei 80 °C innerhalb 2,5 Stunden unter Rühren 545 g (5,5 Mol) Phosgen und rührt nach beendetem Einleiten weitere 45 Minuten bis zum Ende der Kohlendioxid-Entwicklung bei 80 °C nach. Die Reaktionsmischung wird abgekühlt auf 10 °C, das wasserempfindliche Produkt unter Stickstoff abgesaugt, mit 1 l Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76 °C bis 78 °C.

Beispiel 3

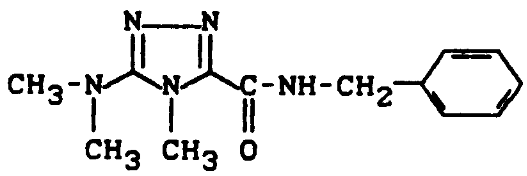

(Verfahren b)

6 g (0.03 Mol) 5-Dimethylamino-4-methyl-4H-1,2,4-triazol-3-yl-carbonsäureethylester und 4,28 g (0.04 Mol) Benzylamin werden in 80 ml Tetrahydrofuran bei 20 bar auf 200 °C erhitzt. Zur Aufarbeitung wird im Vakuum eingeengt und der Rückstand an Kieselgel chromatographisch gereinigt (Laufmittel: Toluol/Ethanol 1:1).

Man erhält 2,68 g (35 % der Theorie) an 5-Dimethylamino-4-methyl-4H-1,2,4-triazol-3-yl-carbonsäure-N-benzylamid vom Schmelzpunkt 96 °C - 98 °C.

Herstellung der Ausgangsverbindung

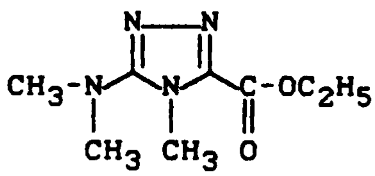

15,25 g (0,1 Mol) 1-Amino-2,2,3-trimethylguanidinium Hydrochlorid und 14,6 g (0,1 Mol) Diethyloxalat werden in 100 ml Dichlorbenzol 8 Stunden bei 180 °C gerührt, anschließend im Vakuum eingeengt und über Kieselgel chromatographiert (Laufmittel: Toluol/Ethanol 1:1).

Man erhält 3,0 g (15 % der Theorie) an 5-Dimethylamino-4-methyl-4H-1,2,4-triazol-3-yl-carbonsäureethylester vom Schmelzpunkt 52 °C - 53 °C.

In entsprechender Weise und gemäß den allgemeinen Angaben zur Herstellung erhält man die folgenden substituierten Triazole der allgemeinen Formel (I):

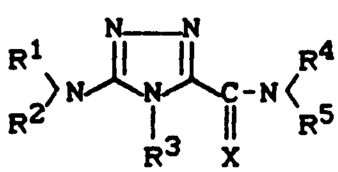

(I)

**Tabelle 1**

| Bsp. Nr. | $R^1$ | $R^2$ | $R^3$ | $R^4$ | $R^5$ | X | physikalische Eigenschaften |
|---|---|---|---|---|---|---|---|
| 4 | $CH_3$ | $CH_3$ | $CH_3$ | H | H | O | Fp 173-174° C |
| 5 | $CH_3$ | $CH_3$ | $CH_3$ | H | 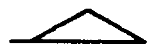 | O | Fp 103-105° C |
| 6 | $CH_3$ | $CH_3$ | $CH_3$ | H | 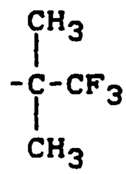 | O | $^1$H-NMR*): 1,7; 7,3 |
| 7 | $CH_3$ | $CH_3$ | $CH_3$ | H | 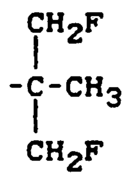 | O | $^1$H-NMR*): 1,5; 4,6 |
| 8 | $CH_3$ | $CH_3$ | $CH_3$ | H | 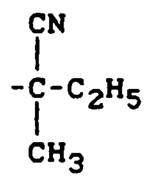 | O | Fp 129-131° C |
| 9 | $CH_3$ | $CH_3$ | $CH_3$ | H | $-C(CH_3)_3$ | O | Fp 64-65° C |
| 10 | $CH_3$ | $CH_3$ | $CH_3$ | H | $-CH_2-C(CH_3)_3$ | O | $^1$H-NMR*): 0,9; 3,2 |
| 11 | $CH_3$ | $CH_3$ | $CH_3$ | H | 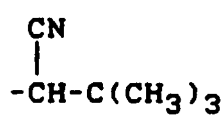 | O | Fp 106-108° C |
| 12 | $CH_3$ | $CH_3$ | $CH_3$ | H | 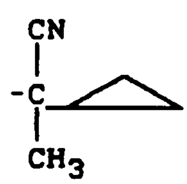 | O | Fp 114-115° C |
| 13 | $CH_3$ | $CH_3$ | $CH_3$ | H | 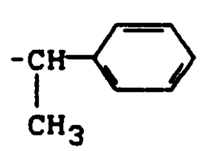 | O | Fp 116-118° C |

<u>Tabelle 1</u> - Fortsetzung

| Bsp. Nr. | $R^1$ | $R^2$ | $R^3$ | $R^4$ | $R^5$ | X | physikalische Eigenschaften |
|---|---|---|---|---|---|---|---|
| 14 | $CH_3$ | $CH_3$ | $CH_3$ | $CH_3$ | $-CH_2-CH_2-CN$ | O | Fp 87-89° C |
| 15 | $CH_3$ | $CH_3$ | $CH_3$ | H | 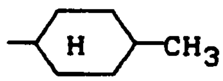 | O | $^1$H-NMR*): 1.0-2.0; 4.1 |
| 16 | $CH_3$ | $CH_3$ | $CH_3$ | H | 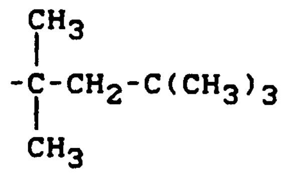 | O | $^1$H-NMR*): 1.0; 1.5; 1.8 |
| 17 | $CH_3$ | $CH_3$ | $CH_3$ | H | $-(CH_2)_5-CH_3$ | O | Fp 30-32° C |
| 18 | $CH_3$ | $CH_3$ | $CH_3$ | H | 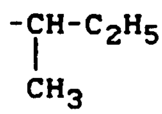 | O | $^1$H-NMR*): 0.9-1.0; 1.2-1.25; 1.5-1.6 |
| 19 | $CH_3$ | $CH_3$ | $CH_3$ | H | $-CH(CH_3)_2$ | O | $^1$H-NMR*): 1.25; 4.1-4.2 |
| 20 | $CH_3$ | $CH_3$ | $CH_3$ | H | $-CH_2-CH_2$ 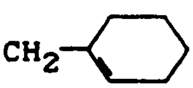 | O | $^1$H-NMR*): 1.5-2.0; 2.3; 3.4; 5.5 |
| 21 | $CH_3$ | $CH_3$ | $CH_3$ | H | 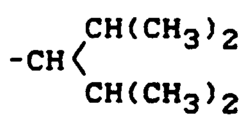 | O | $^1$H-NMR*): 0.9; 1.9; 3.6 |
| 22 | $CH_3$ | $CH_3$ | $CH_3$ | H | 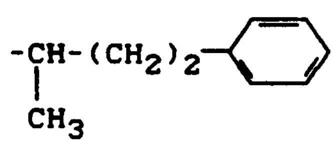 | O | $^1$H-NMR*): 1.3-1.9; 2.7; 4.2; 7.2 |
| 23 | $CH_3$ | $CH_3$ | $CH_3$ | H | 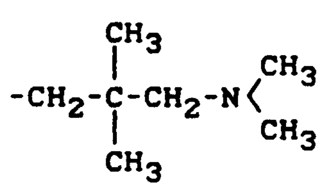 | O | $^1$H-NMR*): 1.0; 2.3; 2.35; 3.3 |

Tabelle 1 - Fortsetzung

| Bsp. Nr. | $R^1$ | $R^2$ | $R^3$ | $R^4$ | $R^5$ | X | physikalische Eigenschaften |
|---|---|---|---|---|---|---|---|
| 24 | $CH_3$ | $CH_3$ | $CH_3$ | H | $-(CH_2)_3-OC_2H_5$ | O | $^1H$-NMR*): 1.2; 1.8; 3.5 |
| 25 | $CH_3$ | $CH_3$ | $CH_3$ | H | $-CH_2-CH_2-Cl$ | O | $^1H$-NMR*): 3.6; 3.7 |
| 26 | $CH_3$ | $CH_3$ | $CH_3$ | H | $-(CH_2)_3-CH_3$ | O | Fp 38-42° C |
| 27 | $CH_3$ | $CH_3$ | $CH_3$ | H | 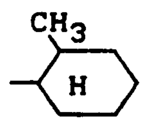 | O | $^1H$-NMR*): 0.9; 1.0-2.0 |
| 28 | $CH_3$ | $CH_3$ | $CH_3$ | H | 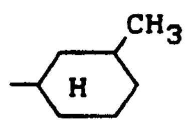 | O | $^1H$-NMR*): 0.9 |
| 29 | $CH_3$ | $CH_3$ | $CH_3$ | H | 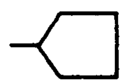 | O | $^1H$-NMR*): |
| 30 | $CH_3$ | $CH_3$ | $CH_3$ | H | $-CH_2-CH(CH_3)_2$ | O | Fp 91-94° C |
| 31 | $CH_3$ | $CH_3$ | $CH_3$ | H | $-CH_2-CH_2-OC_2H_5$ | O | Fp 66-72° C |
| 32 | $CH_3$ | $CH_3$ | $CH_3$ | H | $-(CH_2)_2-CH_3$ | O | Fp 77-78° C |
| 33 | $CH_3$ | $CH_3$ | $CH_3$ | H | 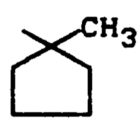 | O | Fp 72-73° C |
| 34 | $CH_3$ | $CH_3$ | $CH_3$ | H | 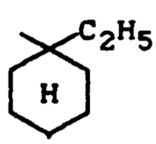 | O | |
| 35 | $CH_3$ | H | $CH_3$ | H | 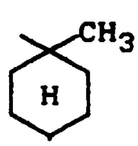 | O | Fp 238-239° C |

Tabelle 1 - Fortsetzung

| Bsp. Nr. | $R^1$ | $R^2$ | $R^3$ | $R^4$ | $R^5$ | X | physikalische Eigenschaften |
|---|---|---|---|---|---|---|---|
| 36 | $CH_3$ | $CH_3$ | $CH_3$ | H | $-CH-CH_2-OCH_3$<br>\|<br>$CH_3$ | O | $^1H$-NMR*): 1.3; 4.3; |
| 37 | $CH_3$ | $CH_3$ | $CH_3$ | H | $-CH-CH_2Cl$<br>\|<br>$CH_3$ | O | $^1H$-NMR*): 1.4; 4.4 |
| 38 | $CH_3$ | $CH_3$ | $CH_3$ | H | $-CH_2-CH-(CH_2)_3-CH_3$<br>\|<br>$C_2H_5$ | O | $^1H$-NMR*): 3.3-3.4 |
| 39 | $CH_3$ | $CH_3$ | $CH_3$ | H | $CH_3$<br>\|<br>$-C-C_2H_5$<br>\|<br>$CH_3$ | O | $^1H$-NMR*): 1.4; |
| 40 | $CH_3$ | $CH_3$ | $CH_3$ | H | $-CH-CH_2-CH(CH_3)_2$<br>\|<br>$CH_3$ | O | $^1H$-NMR*): 4.15 |
| 41 | $CH_3$ | $CH_3$ | $CH_3$ | H | $-(CH_2)_4-$ 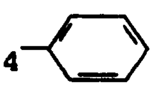 | O | $^1H$-NMR*) 7.1-7.3 |
| 42 | $CH_3$ | $CH_3$ | $CH_3$ | H | $-CH-(CH_2)_4-CH_3$<br>\|<br>$CH_3$ | O | $^1H$-NMR*) 4.1 |
| 43 | $CH_3$ | $CH_3$ | $CH_3$ | H | $-CH-CH(CH_3)_2$<br>\|<br>$CH_3$ | O | $^1H$-NMR*): 1.2;1.7-1.8 |
| 44 | $CH_3$ | $CH_3$ | $CH_3$ | H | $-CH-(CH_2)_3-CH(CH_3)_2$<br>\|<br>$CH_3$ | O | $^1H$-NMR*): 2.1; 4.1 |
| 45 | $CH_3$ | $CH_3$ | $CH_3$ | H | $CH_3$<br>\|<br>$-C-C≡CH$<br>\|<br>$CH_3$ | O | $^1H$-NMR*): 1.75 |

<u>Tabelle 1</u> - Fortsetzung

| Bsp. Nr. | $R^1$ | $R^2$ | $R^3$ | $R^4$ | $R^5$ | X | physikalische Eigenschaften |
|---|---|---|---|---|---|---|---|
| 46 | $CH_3$ | $CH_3$ | $CH_3$ | H | $-CH_2-CH-C_2H_5$<br>$CH_3$ | O | Fp. 40-42° C |
| 47 | $CH_3$ | $CH_3$ | $CH_3$ | H | $-CH-CH_2-CH_2-CH_3$<br>$CH_3$ | O | $^1H$-NMR*): 4.1 |
| 48 | $CH_3$ | $CH_3$ | $CH_3$ | H | $-CH-(CH_2)_3-CH_3$<br>$C_2H_5$ | O | $^1H$-NMR*): 3.95 |
| 49 | $CH_3$ | $CH_3$ | $C_2H_5$ | H | $-CH-C_2H_5$<br>$CH_3$ | O | $^1H$-NMR*): 4.25-4.35 |
| 50 | $CH_3$ | $CH_3$ | $CH_3$ | H | $C_2H_5$<br>$-CH$<br>$C_2H_5$ | O | $^1H$-NMR*): 0.95 |
| 51 | $CH_3$ | $CH_3$ | $C_2H_5$ | H | 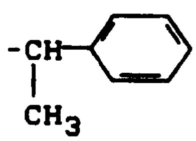 | O | Fp. 121-123° C |
| 52 | $CH_3$ | $CH_3$ | $C_2H_5$ | H | $CH_2F$<br>$-C-CH_3$<br>$CH_2F$ | O | $^1H$-NMR*): 1.35-1.40 |
| 53 | $CH_3$ | $CH_3$ | $C_2H_5$ | H | $-CH(CH_3)_2$ | O | $^1H$-NMR*): 1,4-1,45 |
| 54 | $CH_3$ | $CH_3$ | $CH_3$ | H | $CH_3$<br>$-C-CH(CH_3)_2$<br>$CH_3$ | O | $^1H$-NMR*): 0.9-0.95 |
| 55 | $CH_3$ | $CH_3$ | $CH_3$ | H | $CH_3$ | O | Fp. 95-98° C |

<u>Tabelle 1</u> - Fortsetzung

| Bsp. Nr. | $R^1$ | $R^2$ | $R^3$ | $R^4$ | $R^5$ | X | physikalische Eigenschaften |
|---|---|---|---|---|---|---|---|
| 56 | $CH_3$ | $CH_3$ | $CH_3$ | H | 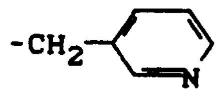 | O | Fp. 117-118° C |
| 57 | $CH_3$ | $CH_3$ | $CH_3$ | H | 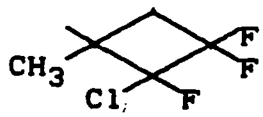 | O | Fp. 155-157° C |
| 58 | $CH_3$ | $CH_3$ | $CH_3$ | H | 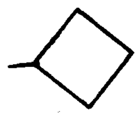 | O | |
| 59 | $CH_3$ | $CH_3$ | $CH_3$ | $C_2H_5$ | $C_2H_5$ | O | |
| 60 | $CH_3$ | $CH_3$ | $CH_3$ | H | 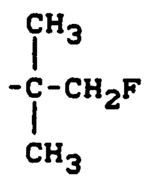 | O | |
| 61 | $CH_3$ | $CH_3$ | $CH_3$ | $-CH(CH_3)_2$ | $-CH(CH_3)_2$ | O | |
| 62 | $C_2H_5$ | $C_2H_5$ | $CH_3$ | H | 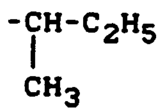 | O | Fp. 57-59° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

<u>Anwendungsbeispiele:</u>

In den folgenden Anwendungsbeispielen wurde die nachstehend aufgeführte Verbindung als Vergleichsubstanz eingesetzt:

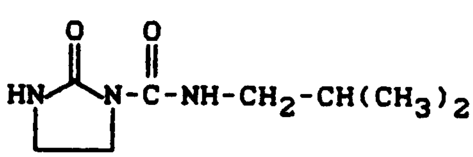

(A)

Imidazolidin-2-on-1-carbonsäureisobutylamid
(bekannt aus K. H. Büchel, "Pflanzenschutz und Schädlingsbekämpfung" S. 170, Thieme Verlag Stuttgart 1977)

Beispiel A

Pre-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung
Eine deutliche Überlegenheit in der Wirksamkeit gegenüber der Vergleichssubstanz (A) zeigen in diesem Test beispielsweise die Verbindungen gemäß folgender Herstellungsbeispiele: 2, 6, 9, 10, 11, 18 und 19.

Beispiel B

Post-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber der Vergleichssubstanz (A) bei vergleichbarer Nutzpflanzenselektivität zeigen in diesem Test beispielsweise die Verbindungen gemäß folgender Herstellungsbeispiele: 2, 7, 10, 11, 13, 14, 15, 18, 19, 22 und 25.

## Patentansprüche

**1.** Substituierte Triazole der Formel (I) gemäß Anspruch 1,

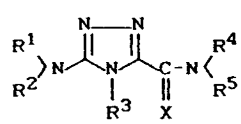

(I)

in welcher

$R^1$ und $R^2$ unabhängig voneinander jeweils für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und

1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im geradkettigen oder verzweigten Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im geradkettigen oder verzweigten Alkylteil, Aryl mit 6 bis 10 Kohlenstoffatomen oder Heteroaryl mit 2 bis 9 Kohlenstoffatomen und 1 bis 3 Heteroatomen stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, enthalten kann, wobei als Substituenten infrage kommen: Halogen, jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo-oder Thionogruppen,

$R^3$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl mit 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^4$ und $R^5$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoximinoalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im geradkettigen oder verzweigten Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen oder jeweils zweifach verknüpftes Alkandiyl bzw. Alkendiyl mit jeweils bis zu 4 Kohlenstoffatomen; außerdem für im Heterocyclylteil gegebenenfalls einfach oder mehrfach, gleich oder verchieden substituiertes Heterocyclylalkyl mit 1 bis 6 Kohlenstoffatomen im geradkettigen oder verzweigten Alkylteil und 1 bis 9 Kohlenstoffatomen sowie 1 bis 3

Heteroatomen im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen und außerdem für jeweils gegebenenfalls einfach oder mehrfach, gleich oder verschieden substituiertes Aralkyl, Aroyl oder Aryl mit jeweils 6 bis 10 Kohlenstoffatomen im Arylteil und gegebenenfalls 1 bis 8 Kohlenstoffatomen im geradkettigen oder verzweigten Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^4$ und $R^5$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome enthalten kann, wobei als Substituenten infrage kommen: Halogen, jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo-oder Thionogruppen und

X für Sauerstoff oder Schwefel steht, ausgenommen die Verbindungen, in denen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alkylteil steht.

**2.** Substituierte Triazole der Formel (I) gemäß Anspruch 1, in welcher

$R^1$ und $R^2$ unabhängig voneinander jeweils für Wasserstoff, für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl stehen, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

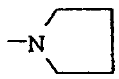 ; 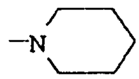 ; 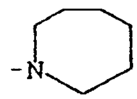 ; 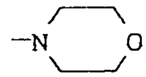 ;

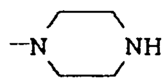 ; 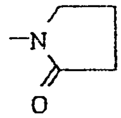 ; 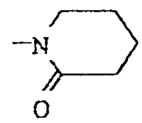 ; 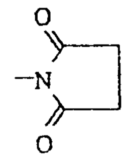 ;

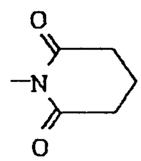 ; 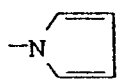 oder 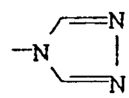 stehen,

wobei als Substituenten jeweils infrage kommen: Methyl,Ethyl, n- oder i-Propyl, Chlor oder Trifluormethyl,

$R^3$ für Methyl, Ethyl, n- oder i-Propyl, n-, i-, s- oder t-Butyl, n- oder i-Pentyl, n- oder i-Hexyl, für Allyl, Propargyl, für Methoxymethyl, für geradkettiges oder verzweigtes Halogenalkyl mit 1 bis 4 Kohlenstoffatomen und 1 bis 9 gleichen oder verschiedenen Halogenatomen, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^4$ und $R^5$ unabhängig voneinander jeweils für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, oder Dodecyl, für Allyl, n- oder i-Butenyl, n- oder i-Pentenyl, n- oder i-Hexenyl, Propargyl, n-oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 5 Kohlenstoffatomen und 1 bis 3 Halogenatomen, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein- bis dreifach, gleich oder verschieden substituiertes Cyclopropyl, Cyclopropylmethyl, Cyclopropylethyl, Cyclobutyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl stehen, wobei als Substituenten jeweils infrage kommen: Fluor, Chlor, Brom, Methyl, Ethyl, n- oder i-Propyl, n-, i-, s-oder t-Butyl, Cyano, Methandiyl, Ethandiyl, Butandiyl oder Butadiendiyl;

außerdem für im Heterocyclylteil gegebenenfalls ein- bis dreifach, gleich oder verschieden substituiertes Heterocyclylmethyl, Heterocyclylpropyl oder Heterocyclylethyl stehen, wobei als Heterocyclen jeweils infrage kommen:

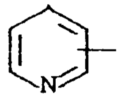 ; 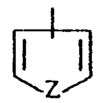 ; 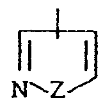 ; 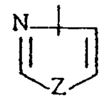 ;

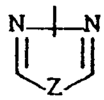 ; 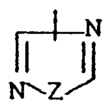 ; 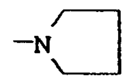 ; 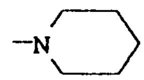 ;

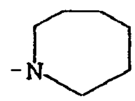 ; 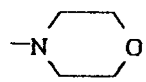 oder 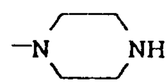 ,

wobei Z jeweils für Sauerstoff oder Schwefel steht und wobei als Substituenten jeweils infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;
außerdem für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy oder

$R^4$ und $R^5$ gemeinsam mit dem Stickstoffatom, an welches sie gebunden sind, für einen gegebenenfalls ein- bis dreifach, gleich oder verschieden substituierten Heterocyclus der Formel

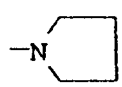 ; 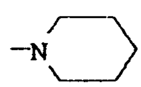 ; 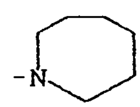 ; 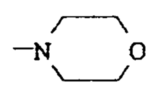 ;

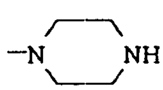 ; 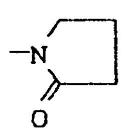 ; 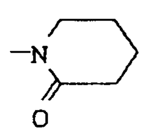 ; 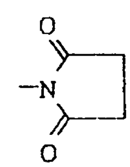 ;

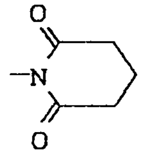 ; 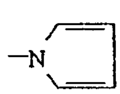 oder 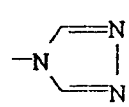 stehen,

wobei als Substituenten jeweils infrage kommen: Methyl, Ethyl, n- oder i-Propyl, Chlor oder Trifluormethyl und

| | |
|---|---|
| X | für Sauerstoff oder Schwefel steht, ausgenommen die Verbindungen, in denen |
| $R^1$ und $R^2$ | gemeinsam mit dem Stickstoffatom, an welches sie gebunden sind, für einen gegebenenfalls substituierten Heterocyclus stehen, |
| X | für Schwefel steht, |
| $R^3$ | für Aryl mit 6 bis 10 Kohlenstoffatomen steht, |
| $R^4$ und $R^5$ | unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alkylteil steht. |

**3.** Substituierte Triazole der Formel (I) gemäß Anspruch 1, in welcher

| | |
|---|---|
| $R^1$ | für Wasserstoff, Methyl oder Ethyl steht, |
| $R^2$ | für Methyl oder Ethyl steht, |
| $R^3$ | für Methyl oder Ethyl steht, |
| $R^4$ | für Wasserstoff oder Methyl steht, |
| $R^5$ | für Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 5 Kohlenstoffatomen und 1 bis 3 Halogenatomen, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl-bzw. Alkenylteilen oder für jeweils gegebenenfalls ein- bis dreifach, gleich oder verschieden durch Fluor, Chlor, Brom, Methyl, Ethyl, n-oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclobutyl Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht; |

außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen infrage kommen:

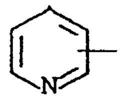 ; 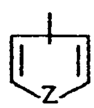 ; 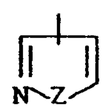 ; 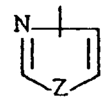 ;

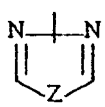 ; 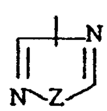 ; 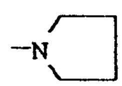 ; 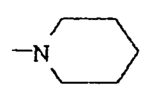 ;

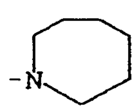 ; 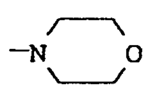 oder 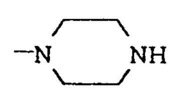 ,

wobei Z jeweils für Sauerstoff oder Schwefel steht,

außerdem für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht und

X für Sauerstoff steht, ausgenommen die Verbindungen, in denen

$R^1$ und $R^2$ gemeinsam mit dem Stickstoffatom, anwelches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alkylteil steht.

**4.** Verfahren zur Herstellung von substituierten Triazolen der Formel (I),

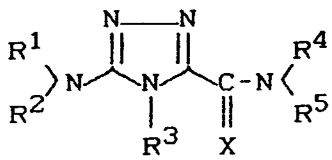

(I)

in welcher

$R^1$, $R^2$, $R^3$, $R^4$, R5 und X die in Anspruch 1 genannte Definition haben, ausgenommen die Verbindungen

$R^1$ und $R^2$ gemeinsam mit dem Stickstoffatom, an welches sie gebunden sind, für einen gegebenenfalls substituierten Heterocyclus stehen,

X für Schwefel steht,

$R^3$ für Aryl mit 6 bis 10 Kohlenstoffatomen steht,

$R^4$ und $R^5$ unabhängig voneinander jeweils für Wasserstoff, für jeweils geradkettiges oder verzweigtes Alkyl mit 1 bis 18 Kohlenstoffatomen steht, außerdem für gegebenenfalls einfach oder mehrfach, gleich oder verschieden substituiertes Aryl oder Aralkyl mit jeweils 6 bis 10 Kohlenstoffatomen im Arylteil und gegebenenfalls 1 bis 8 Kohlenstoffatomen im geradkettigen oder verzweigten Alkylteil steht,

dadurch gekennzeichnet, daß man

(a) Aminoguanidine der Formel (II),

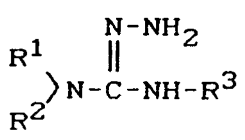

(II)

in welcher

$R^1$, $R^2$ und $R^3$ die oben angegebene Bedeutung haben,

oder deren Säureadditionssalze mit (Thio)-Oxalesteramiden der Formel (III),

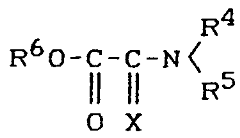

(III)

in welcher

$R^6$ für Alkyl steht und

$R^4$, $R^5$ und X die oben angegebene Bedeutung haben,

gegebenenfalls in Gegenwart eines Verdünnungsmittels und gegebenenfalls in Gegenwart eines Reaktionshilfsmittels umsetzt oder daß man

(b) substituierte Triazolyl(thio)carbonsäureester der Formel (IV),

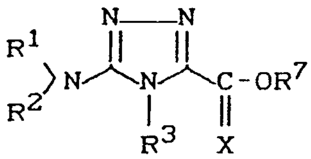

(IV)

in welcher

$R^7$ für Alkyl steht und

$R^1$, $R^2$, $R^3$ und X die oben angegebene Bedeutung haben,

mit Aminen der Formel (V),

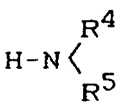

(V)

in welcher

$R^4$ und $R^5$ die oben angegebene Bedeutung haben,

gegebenenfalls in Gegenwart eines Verdünnungsmittels umsetzt.

**5.** Herbizide Mittel, gekennzeichnet durch einen Gehalt an mindestens einem substituierten Triazol der Formel (I) gemäß den Ansprüchen 1 bis 4.

**6.** Verfahren zur Bekämpfung von Unkräutern, dadurch gekennzeichnet, daß man substituierte Triazole der Formel (I) gemäß den Ansprüchen 1 bis 4 auf die Unkräuter und/oder ihren Lebensraum einwirken laßt.

**7.** Verwendung von substituierten Triazolen der Formel (I) gemäß den Ansprüchen 1 bis 4 zur Bekämpfung von Unkräutern.

**8.** Verfahren zur Herstellung von herbiziden Mitteln, dadurch gekennzeichnet, daß man substituierte Triazole der Formel (I) gemäß den Ansprüchen 1 bis 4 mit Streckmitteln und/oder oberflächenaktiven Substanzen vermischt.

**9.** Die Verbindungen der Formeln

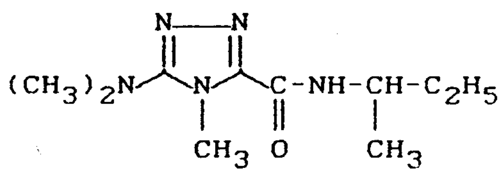

und

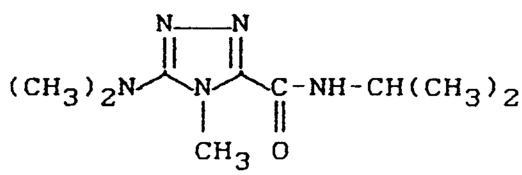

## Claims

**1.** Substituted triazoles of the formula (I)

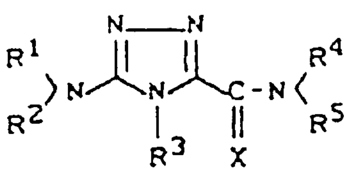

(I)

in which

$R^1$ and $R^2$ independently of one another each represent hydrogen, in each case straight-chain or branched alkyl which has 1 to 8 carbon atoms, alkenyl which has 2 to 8 carbon atoms, alkinyl which has 2 to 8 carbon atoms, alkinyl which has 2 to 8 carbon atoms, halogenoalkyl which has 1 to 8 carbon atoms and 1 to 17 identical or different halogen atoms, halogenoalkenyl or halogenoalkinyl, each of which has 2 to 8 carbon atoms and 1 to 15 or 13 identical or different halogen atoms respectively, alkoxyalkyl which has 1 to 6 carbon atoms in the individual alkyl moieties, cycloalkyl which has 3 to 7 carbon atoms, cycloalkylalkyl which has 3 to 7 carbon atoms in the cycloalkyl moiety and 1 to 6 carbon atoms in the straight-chain or branched alkyl moiety or represent aralkyl which has 6 to 10 carbon atoms in the aryl moiety and 1 to 6 carbon atoms in the straight-chain or branched alkyl moiety, aryl which has 6 to 10 carbon atoms or heteroaryl which has 2 to 9 carbon atoms and 1 to 3 hetero atoms, each of which is optionally substituted once or more than once by identical or different substituents from the series comprising halogen, cyano, nitro and also in each case straight-chain or branched alkyl, alkoxy, alkylthio, halogenoalkyl, halogenoalkoxy or halogenoalkylthio, each of which has 1 to 4 carbon atoms and if appropriate 1 to 9 identical or different halogen atoms, or

$R^1$ and $R^2$ together with the nitrogen atom to which they are bonded represent a five- to ten-membered heterocyclic ring which may contain, if appropriate, 1 to 2 other hetero atoms, and which is optionally substituted once or more than once by identical or different substituents, suitable substituents being: halogen and also in each case straight-chain or branched alkyl or halogenoalkyl, each of which has 1 to 4 carbon atoms and if appropriate 1 to 9 identical or different halogen atoms and also 1 to 2 oxo or thiono groups,

$R^3$ represents in each case straight-chain or branched alkyl which has 1 to 8 carbon atoms, alkenyl which has 2 to 8 carbon atoms, alkinyl which has 2 to 8 carbon atoms, halogenoalkyl which has 1 to 8 carbon atoms and 1 to 17 identical or different halogen atoms, halogenoalkenyl which has 2 to 8 carbon atoms and 1 to 15 identical or different halogen atoms, halogenoalkinyl which has 2 to 8 carbon atoms and 1 to 13 identical or different halogen atoms, alkoxyalkyl which has 1 to 6 carbon atoms in each of the individual alkyl moieties, cycloalkylalkyl or cycloalkyl each of which has 3 to 7 carbon atoms in the cycloalkyl moiety and if appropriate 1 to 6 carbon atoms in the straight-chain or branched alkyl moiety, or represents aralkyl or aryl, each of which has 6 to 10 carbon atoms in the aryl moiety and if appropriate 1 to 6 carbon atoms in the straight-chain or branched alkyl moiety and each of which is optionally substituted once or more than once by identical or different substituents, suitable aryl substituents in each case being: halogen, cyano, nitro and also in each case straight-chain or branched alkyl, alkoxy, alkylthio, halogenoalkyl, halogenoalkoxy or halogenoalkylthio, each of which has 1 to 4 carbon atoms and if appropriate 1 to 9 identical or different halogen atoms,

$R^4$ and $R^5$ independently of one another each represent hydrogen, in each case straight-chain or branched alkyl which has 1 to 18 carbon atoms, alkenyl which has 2 to 8 carbon atoms, alkinyl which has 2 to 8 carbon atoms, halogenoalkyl which has 1 to 8 carbon atoms and 1 to 17 identical or different halogen atoms, halogenoalkenyl or halogenoalkinyl, each of which has 2 to 8 carbon atoms and 1 to 15, or 13, identical or different halogen atoms, respectively, cyanoalkyl which has 1 to 8 carbon atoms, hydroxyalkyl which has 1 to 8 carbon atoms and 1 to 6 hydroxyl groups, alkoxyalkyl, alkoximinoalkyl, alkoxycarbonylalkyl or alkoxycarbonylalkenyl, each of which has up to 6 carbon atoms in the individual alkyl moieties, or alkenyl moieties, respectively, alkylaminoalkyl or dialkylaminoalkyl, each of which has 1 to 6 carbon atoms in the individual alkyl moieties, or represent cycloalkyl, cycloalkylalkyl, cycloalkenyl or cycloalkenylalkyl, each of which has 3 to 8 carbon atoms in the cycloalkyl moiety, or cycloalkenyl moiety, respectively, and if appropriate 1 to 6 carbon atoms in the straight-chain or branched alkyl moiety and each of which is optionally substituted once or more than once by identical or different substituents from the series comprising halogen, cyano and in each case straight-chain or branched alkyl or halogenoalkyl, each of which has 1 to 4 carbon atoms and if appropriate 1 to 9 identical or different halogen atoms or in each case double-linked alkanediyl, or alkenediyl, each of which has up to 4 carbon atoms; furthermore represent heterocyclylalkyl which has 1 to 6 carbon atoms in the straight-chain or branched alkyl moiety and 1 to 9 carbon atoms and also 1 to 3 hetero atoms in the heterocyclyl moiety and which is optionally substituted once or more than once in the heterocyclyl moiety by identical or different substituents, suitable substituents being: halogen, cyano, nitro, and also in each case straight-chain or branched alkyl, alkoxy, alkylthio, halogenoalkyl, halogenoalkoxy, halogenoalkylthio or alkoxycarbonyl, each of which has 1 to 5 carbon atoms and if appropriate 1 to 9 identical or different halogen atoms, and, in addition, represent aralkyl, aroyl or aryl, each of which has 6 to 10 carbon atoms in the aryl moiety and if appropriate 1 to 8 carbon atoms in the straight-chain or branched alkyl moiety and each of which is optionally substituted once or more than once by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms and if appropriate 1 to 9 identical or different halogen atoms, cycloalkyl which has 3 to 6 carbon atoms or phenoxy, and suitable alkyl substituents, if appropriate, being: halogen or cyano, or

$R^4$ and $R^5$ together with the nitrogen atom to which they are bonded represent a five- to ten-membered heterocyclic ring which may contain, if appropriate, 1 to 2 more hetero atoms, and which is optionally substituted once or more than once by identical or different substituents, suitable substituents being: halogen and also in each case straight-chain or branched alkyl or halogenoalkyl, each of which has 1 to 4 carbon atoms and if appropriate 1 to 9 identical or different halogen atoms and also 1 to 2

oxo or thiono groups, and

X represents oxygen or sulphur, with the exception of the compounds in which

$R^1$ and $R^2$ together with the nitrogen atom to which they are bonded represent an optionally substituted heterocycle,

X represents sulphur,

$R^3$ represents aryl having 6 to 10 carbon atoms,

$R^4$ and $R^5$ independently of one another in each case represent hydrogen, in each case straight-chain or branched alkyl having 1 to 18 carbon atoms, and furthermore represent aryl or aralkyl, each of which has 6 to 10 carbon atoms in the aryl moiety and, if appropriate, 1 to 8 carbon atoms in the straight-chain or branched alkyl moiety and is optionally monosubstituted or polysubstituted by identical or different substituents.

**2.** Substituted triazoles of the formula (I) according to Claim 1, in which

$R^1$ and $R^2$ independently of one another each represent hydrogen, methyl, ethyl, n- or i-propyl, n-, i-, s- or t-butyl, n- or i-pentyl, allyl, propargyl, in each case straight-chain or branched halogenoalkyl which has 1 to 4 carbon atoms, halogenoalkenyl which has 3 to 6 carbon atoms or halogenoalkinyl which has 3 to 6 carbon atoms, each of which has 1 to 9 identical or different halogen atoms, methoxymethyl, methoxyethyl, cyclopropyl, cyclopropylmethyl, cyclopentyl, cyclohexyl, cyclohexylmethyl, cyclohexylethyl, cyclopentylmethyl or represent benzyl, phenylethyl or phenyl, each of which is optionally substituted once, to three times by identical or different substituents, suitable substituents being in each case: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio, or

$R^1$ and $R^2$ together with the nitrogen atom to which they are bonded represent a heterocyclic ring of the formula

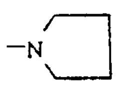 ; 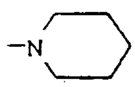 ; 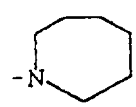 ; 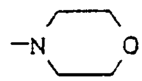 ;

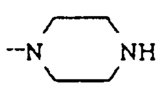 ; 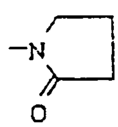 ; 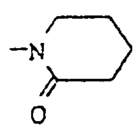 ; 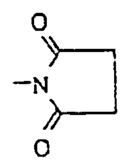 ;

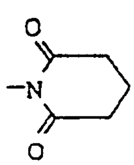 ; 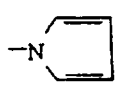 or 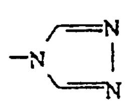

each of which is optionally substituted once to three times by identical or different substituents, suitable substituents in each case being: methyl, ethyl, n- or i-propyl, chlorine or trifluoromethyl,

$R^3$ represents methyl, ethyl, n- or i-propyl, n-, i-, s- or t-butyl, n- or i-pentyl, n- or i-hexyl, allyl, propargyl, methoxymethyl, straight-chain or branched halogenoalkyl which has 1 to 4 carbon atoms and 1 to 9 identical or different halogen atoms, cyclopentyl, cyclohexyl, cyclopropyl, cyclopropylmethyl, cyclohexylmethyl, cyclohexylethyl or represents benzyl or phenyl, each of which is optionally substituted once to three times by identical or different substituents, suitable substituents in each case being: fluorine, chlorine, bromine, cyano, nitro, methyl, ethyl, n- or i-propyl, n-, i-, s- or t-butyl,

methoxy, ethoxy, methylthio, trifluoromethyl, trifluoromethoxy or trifluoromethylthio,

$R^4$ and $R^5$ independently of one another each represent hydrogen, methyl, ethyl, n- or i-propyl, n-, i-, s- or t-butyl, in each case straight-chain or branched pentyl, hexyl, heptyl, octyl, nonyl, decyl or dodecyl, or represent allyl, n- or i-butenyl, n- or i-pentenyl, n- or i-hexenyl, propargyl, n- or i-butinyl, n- or i-pentinyl, n- or i-hexinyl, straight-chain or branched halogenoalkyl which has 1 to 6 carbon atoms and 1 to 9 identical or different halogen atoms, in each case straight-chain or branched halogenoalkenyl or halogenoalkinyl, each of which has 3 to 5 carbon atoms and 1 to 3 halogen atoms, in each case straight-chain or branched cyanoalkyl which has 1 to 6 carbon atoms in the alkyl moiety, hydroxyalkyl which has 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, each of which has up to 4 carbon atoms in the individual alkyl moieties, or alkenyl moieties, respectively, or represent cyclopropyl, cyclopropylmethyl, cyclopropylethyl, cyclobutyl, cyclopentyl, cyclohexyl, cycloheptyl, cyclooctyl, cyclohexylmethyl, cyclohexylethyl, cyclohexenyl, cyclohexenylmethyl or cyclohexenylethyl, each of which is optionally substituted once to three times by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl;

additionally represent heterocyclylmethyl, heterocyclylpropyl or heterocyclylethyl, each of which is optionally substituted once to three times in the heterocyclyl moiety by identical or different substituents, suitable heterocyclic rings in each case being:

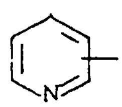 ; 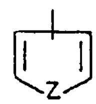 ; 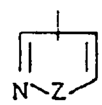 ; 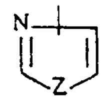 ;

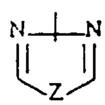 ; 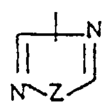 ; 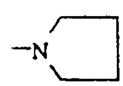 ; 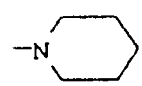 ;

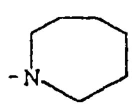 ; 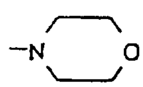 or 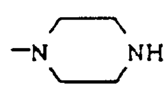 ,

where Z in each case represents oxygen or sulphur, and suitable substituents in each case being:

fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

additionally represent optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzoyl, phenyl or naphthyl, each of which is optionally substituted once to three times by identical or different substituents suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, or

$R^4$ and $R^5$ together with the nitrogen atom to which they are bonded represent a heterocyclic ring of the formula

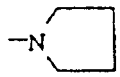 ; 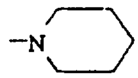 ; 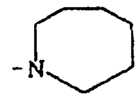 ; 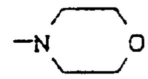 ;

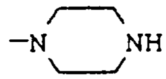 ; 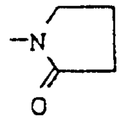 ; 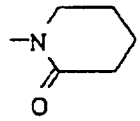 ; 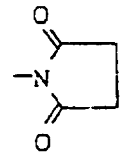 ;

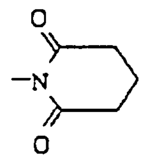 ; 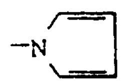 or 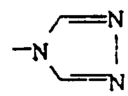 .

each of which is optionally substituted once to three times by identical or different substituents, suitable substituents in each case being: methyl, ethyl, n- or i-propyl, chlorine or trifluoromethyl, and

X represents oxygen or sulphur, with the exception of the compounds in which

$R^1$ and $R^2$ together with the nitrogen atom to which they are bonded represent an optionally substituted heterocycle,

X represents sulphur,

$R^3$ represents aryl having 6 to 10 carbon atoms,

$R^4$ and $R^5$ independently of one another in each case represent hydrogen, in each case straight-chain or branched alkyl having 1 to 18 carbon atoms, and furthermore represent aryl or aralkyl, each of which have 6 to 10 carbon atoms in the aryl moiety and, if appropriate, 1 to 8 carbon atoms in the straight-chain or branched alkyl moiety and is optionally monosubstituted or polysubstituted by identical or different substituents.

**3.** Substituted triazoles of the formula (I) according to Claim 1, in which

$R^1$ represents hydrogen, methyl or ethyl,

$R^2$ represents methyl or ethyl,

$R^3$ represents methyl or ethyl,

$R^4$ represents hydrogen or methyl,

$R^5$ represents hydrogen, methyl, ethyl, n- or i-propyl, n-, i-, s- or t-butyl, in each case straight-chain or branched pentyl, hexyl, heptyl, octyl, nonyl, decyl or dodecyl, or represents allyl, n- or i-butenyl, n- or i-pentenyl, n- or i-hexenyl, propargyl, n- or i-butinyl, n- or i-pentinyl, n- or i-hexinyl, straight-chain or branched halogenoalkyl which has 1 to 6 carbon atoms and 1 to 9 identical or different halogen atoms, in each case straight-chain or branched halogenoalkenyl or halogenoalkinyl, each of which has 3 to 5 carbon atoms and 1 to 3 halogen atoms, in each case straight-chain or branched cyanoalkyl which has 1 to 6 carbon atoms in the alkyl moiety, hydroxyalkyl which has 1 to 6 carbon atoms and 1 to 3 hydroxyl groups, alkoxyalkyl, alkoximinoalkyl, alkoxycarbonylalkyl or alkoxycarbonylalkenyl, alkylaminoalkyl or dialkylaminoalkyl, each of which has up to 4 carbon atoms in the individual alkyl moieties, or alkenyl moieties, respectively, or represents cyclopropyl, cyclopropylmethyl, cyclopropylethyl, cyclobutyl, cyclopentyl, cyclohexyl, cycloheptyl, cyclooctyl, cyclohexylmethyl, cyclohexylethyl, cyclohexenyl, cyclohexenylmethyl or cyclohexenylethyl, each of which is optionally substituted once to three times by identical or different substituents from the series comprising fluorine, chlorine, bromine, methyl, ethyl, n- or i-propyl, n-, i-, s- or t-butyl, cyano, methanediyl, ethanediyl, butanediyl or butadienediyl;

additionally represents heterocyclylmethyl, heterocyclylpropyl or heterocyclylethyl,

each of which is optionally substituted once to three times in the heterocyclyl moiety by identical or different substituents from the series comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio, suitable heterocyclic rings being:

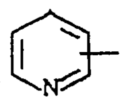 ; 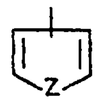 ; 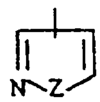 ; 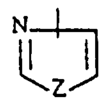 ;

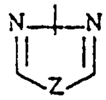 ; 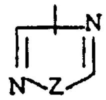 ; 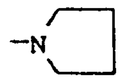 ; 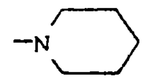 ;

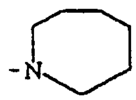 ; 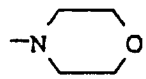 or 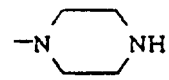 ,

with Z in each case representing oxygen or sulphur,
additionally represents in each case straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzoyl, phenyl or naphthyl, each of which is optionally substituted once to three times in the phenyl moiety by identical or different substituents from the series comprising fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, and

| | |
|---|---|
| X | represents oxygen, with the exception of the compounds in which |
| $R^1$ and $R^2$ | together with the nitrogen atom to which they are bonded represent an optionally substituted heterocycle, |
| X | represents sulphur, |
| $R^3$ | represents aryl having 6 to 10 carbon atoms, |
| $R^4$ and $R^5$ | independently of one another in each case represent hydrogen, in each case straight-chain or branched alkyl having 1 to 18 carbon atoms, and furthermore represent aryl or aralkyl, each of which has 6 to 10 carbon atoms in the aryl moiety and, if appropriate, 1 to 8 carbon atoms in the straight-chain or branched alkyl moiety and is optionally monosubstituted or polysubstituted by identical or different substituents. |

**4.** Process for the preparation of substituted triazoles of the formula (I)

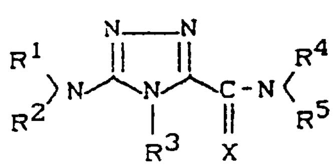 (I)

in which

| | |
|---|---|
| $R^1$, $R^2$, $R^3$, $R^4$, $R^5$ and X | have the definition given in Claim 1, with the exception of the compounds in which |
| $R^1$ and $R^2$ | together with the nitrogen atom to which they are bonded represent an optionally substituted heterocycle, |
| X | represents sulphur, |
| $R^3$ | represents aryl having 6 to 10 carbon atoms, |

$R^4$ and $R^5$ independently of one another in each case represent hydrogen, in each case straight-chain or branched alkyl having 1 to 18 carbon atoms, and furthermore represent aryl or aralkyl, each of which have 6 to 10 carbon atoms in the aryl moiety and, if appropriate, 1 to 8 carbon atoms in the straight-chain or branched alkyl moiety and is optionally monosubstituted or polysubstituted by identical or different substituents,

characterised in that

(a) aminoguanidines of the formula (II)

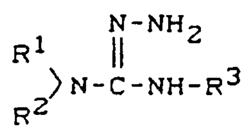 (II)

in which

$R^1$, $R^2$ and $R^3$ have the abovementioned meaning, or their acid addition salts with (thio)oxalic ester amides of the formula (III)

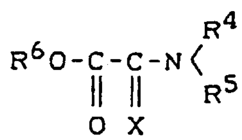 (III)

in which

$R^6$ represents alkyl and

$R^4$, $R^5$ and X have the abovementioned meaning

are reacted if appropriate in the presence of a diluent and if appropriate in the presence of a reaction auxiliary, or in that

(b) substituted triazolyl(thio)carboxylic acid esters of the formula (IV)

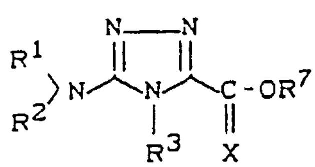 (IV)

in which

$R^7$ represents alkyl and

$R^1$, $R^2$, $R^3$ and X have the abovementioned meaning,

are reacted with amines of the formula (V)

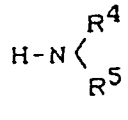 (V)

in which

$R^4$ and $R^5$ have the abovementioned meaning,

if appropriate in the presence of a diluent.

**5.** Herbicidal agents, characterised in that they contain at least one substituted triazole of the formula (I) according to Claims 1 to 4.

**6.** Method of combating weeds, characterised in that substituted triazoles of the formula (I) according to Claims 1 to 4 are allowed to act on weeds and/or their environment.

**7.** Use of substituted triazoles of the formula (I) according to Claims 1 to 4 for combating weeds.

**8.** Process for the preparation of herbicidal agents, characterised in that substituted triazoles of the formula (I) according to Claims 1 to 4 are mixed with extenders and/or surface-active substances.

**9.** The compounds of the formulae

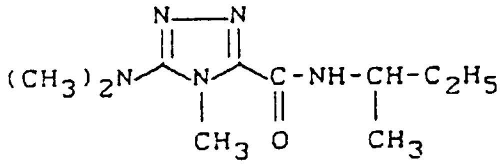

and

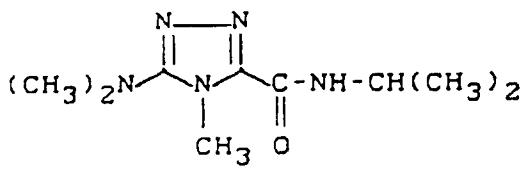

## Revendications

**1.** Triazoles substitués de formule (I) :

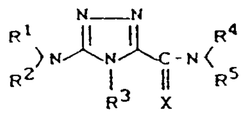

(I)

dans laquelle

$R^1$ et $R^2$ représentent chacun, indépendamment l'un de l'autre, un atome d'hydrogène, un groupe, à chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ou halogéno-alcynyle ayant chacun 2 à 8 atomes de carbone et 1 à 15 ou 13 atomes d'halogène identiques ou différents, alcoxyalkyle ayant 1 à 6 atomes de carbone dans les diverses parties alkyles, un groupe cycloalkyle ayant 3 à 7 atomes de carbone, un groupe cycloalkylalkyle ayant 3 à 7 atomes de carbone dans la partie cycloalkyle et 1 à 6 atomes de carbone dans la partie alkyle linéaire ou ramifiée, ou un groupe, à chaque fois éventuellement substitué une ou plusieurs fois de manière identique ou différente, aralkyle ayant 6 à 10 atomes de carbone dans la partie aryle et 1 à 6 atomes de carbone dans la partie alkyle linéaire ou ramifiée, aryle ayant 6 à 10 atomes de carbone ou hétéroaryle ayant 2 à 9 atomes de carbone et 1 à 3 hétéroatomes, et l'on peut citer à chaque fois comme substituant : un atome d'halogène, un reste cyano, nitro, ainsi qu'un reste, chaque fois linéaire ou ramifié, alkyle, alcoxy, alkylthio, halogéno-alkyle, halogéno-alcoxy ou halogéno-alkylthio, ayant chacun 1 à 4 atomes de carbone et éventuellement 1 à 9

atomes d'halogène, identiques ou différents, ou bien

$R^1$ et $R^2$ forment, avec l'atome d'azote auxquels ils sont reliés, un hétérocycle pentagonal à décagonal, éventuellement substitués une ou plusieurs fois de manière identique ou différente, qui peut éventuellement contenir 1 à 2 autres hétéroatomes, et l'on peut alors citer comme substituants : un atome d'halogène, un reste, chaque fois linéaire ou ramifié, alkyle ou halogéno-alkyle ayant chacun 1 à 4 atomes de carbone et éventuellement 1 à 9 atomes d'halogène, identiques ou différents, ainsi que 1 à 2 groupes oxo ou thiono;

$R^3$ représente un groupe, à chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ayant 2 à 8 atomes de carbone et 1 à 15 atomes d'halogène identiques ou différents, halogéno-alcynyle ayant 2 à 8 atomes de carbone et 1 à 13 atomes d'halogène identiques ou différents, alcoxyalkyle ayant 1 à 6 atomes de carbone dans les diverses parties alkyles, un groupe cycloalkylalkyle ou cycloalkyle ayant chacun 3 à 7 atomes de carbone dans la partie cycloalkyle et éventuellement 1 à 6 atomes de carbone dans la partie alkyle linéaire ou ramifiée, ou bien un groupe, chaque fois éventuellement substitué une ou plusieurs fois, de façon identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer comme substituants du noyau aryle, à chaque fois, un atome d'halogène, un reste cyano, nitro ainsi qu'un reste, chaque fois linéaire ou ramifié, alkyle, alcoxy, alkylthio, halogéno-alkyle, halogéno-alcoxy ou halogéno-alkylthio, ayant chacun 1 à 4 atomes de carbone et éventuellement 1 à 9 atomes d'halogène identiques ou différents,

$R^4$ et $R^5$ représentent chacun, indépendamment l'un de l'autre, un atome d'hydrogène, un groupe, chaque fois linéaire ou ramifié, alkyle ayant 1 à 18 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ou halogéno-alcynyle ayant chaque fois 2 à 8 atomes de carbone et 1 à 15 ou 13 atomes d'halogène identiques ou différents, cyanalkyle ayant 1 à 8 atomes de carbone, hydroxyalkyle ayant 1 à 8 atomes de carbone et 1 à 6 groupes hydroxyle, alcoxyalkyle, alcoxyminoalkyle, alcoxycarbonylalkyle ou alcoxycarbonylalcényle ayant chacun jusqu'à 6 atomes de carbone dans les diverses parties alkyle ou alcényle, alkylaminoalkyle ou dialkylamino-alkyle ayant chaque fois 1 à 6 atomes de carbone dans les diverses parties alkyles ou un groupe, éventuellement substitué une ou plusieurs fois de façon identique ou différente, cycloalkyle, cycloalkylalkyle, cycloalcényle ou cycloalcénylalkyle, ayant à chaque fois 3 à 8 atomes de carbone dans la partie cycloalkyle ou cycloalcényle et éventuellement 1 à 6 atomes de carbone dans la partie alkyle linéaire ou ramifiée, et l'on peut citer comme substituants, à chaque fois, un atome d'halogène, un reste cyano ainsi qu'un reste, à chaque fois linéaire ou ramifié, alkyle ou halogéno-alkyle ayant chacun 1 à 4 atomes de carbone et éventuellement 1 à 9 atomes d'halogène identiques ou différents ou un reste alcanediyle ou alcènediyle, chaque fois doublement liés et comportant chacun jusqu'à 4 atomes de carbone ; en outre, un groupe hétérocyclylalkyle, éventuellement substitué une ou plusieurs fois, de façon identique ou différente dans la partie hétérocyclyle et comportant 1 à 6 atomes de carbone dans la partie alkyle linéaire ou ramifiée et 1 à 9 atomes de carbone ainsi que 1 à 3 hétéroatomes dans la partie hétérocyclyle, et l'on peut citer comme substituant : un atome d'halogène, un reste cyano, nitro, ainsi qu'un reste, à chaque fois linéaire ou ramifié, alkyle, alcoxy, alkylthio, halogéno-alkyle, halogéno-alcoxy, halogéno-alkylthio ou alcoxycarbonyle, ayant chacun 1 à 5 atomes de carbone et éventuellement 1 à 9 atomes d'halogène identiques ou différents et, en outre, un groupe, chaque fois éventuellement substitué une ou plusieurs fois de façon identique ou différente aralkyle, aroyle ou aryle, ayant chacun 6 à 10 atomes de carbone dans la partie aryle et éventuellement 1 à 8 atomes de carbone dans la partie alkyle linéaire ou ramifiée, et l'on peut citer comme substituant du noyau aryle, à chaque fois : un atome d'halogène, un reste cyano, nitro, hydroxy, un reste, à chaque fois linéaire ou ramifié, alkyle, alcoxy, alkylthio, halogéno-alkyle,

halogéno-alcoxy, halogéno-alkylthio, alkylsulfinyle, alkylsulfonyle, halogéno-alkylsulfinyle, halogéno-alkylsulfonyle, alcanoyle ou alcoxycarbonyle, ayant chacun 1 à 6 atomes de carbone et éventuellement 1 à 9 atomes d'halogène identiques ou différents, cycloalkyle ayant 3 à 6 atomes de carbone ou phénoxy, et l'on peut citer comme substituant de la partie alkyle éventuellement : un atome d'halogène ou un reste cyano, ou bien

$R^4$ et $R^5$ forment, avec l'atome d'azote auquel ils sont reliés, un hétérocyle éventuellement substitués une ou plusieurs fois de façon identique ou différente, qui est pentagonal à décagonal et qui peut éventuellement contenir 1 à 2 autres hétéroatomes, et l'on peut citer comme substituant : un atome d'halogène, un reste, chaque fois linéaire ou ramifié, alkyle ou halogéno-alkyle ayant à chaque fois 1 à 4 atomes de carbone et éventuellement 1 à 9 atomes d'halogène identiques ou différents, ainsi que 1 à 2 groupes oxo ou thiono ; et

X représente un atome d'oxygène ou de soufre, à l'exclusion des composés dans lesquels :

$R^1$ et $R^2$, pris avec l'atome d'azote auquel ils sont fixés, représentent un hétérocycle éventuellement substitué,

X représente un atome de soufre,

$R^3$ représente un groupe aryle ayant 6 à 10 atomes de carbone,

$R^4$ et $R^5$ représentent chacun, indépendamment l'un de l'autre, un atome d'hydrogène, un groupe alkyle, chaque fois linéaire ou ramifié et ayant 1 à 18 atomes de carbone, et, en outre, un groupe aryle ou aralkyle, éventuellement substitué une ou plusieurs fois de façon identique ou différente et comportant chacun 6 à 10 atomes de carbone dans la partie aryle et éventuellement 1 à 8 atomes de carbone dans la partie alkyle linéaire ou ramifiée.

**2.** Triazoles substitués de formule (I) selon la revendication 1, dans laquelle,

$R^1$ et $R^2$ représentent chacun, indépendamment l'un de l'autre, un atome d'hydrogène, un groupe méthyle, éthyle, n- ou i-propyle, n-, i-, s- ou t-butyle, n- ou i-pentyle, allyle, propargyle, un groupe, à chaque fois linéaire ou ramifié, halogéno-alkyle ayant 1 à 4 atomes de carbone, halogéno-alcényle ayant 3 0 6 atomes de carbone ou halogéno-alcynyle ayant 3 à 6 atomes de carbone et comportant chacun 1 à 9 atomes d'halogène identiques ou différents, un groupe méthoxyméthyle, méthoxyéthyle, cyclopropyle, cyclopropylméthyle, cyclopentyle, cyclohexyle, cyclohexylméthyle, cyclohexyléthyle, cyclopentylméthyle ou un groupe, à chaque fois éventuellement substitué 1 à 3 fois de façon identique ou différente, benzyle, phényléthyle ou phényle, et l'on peut citer à chaque fois comme substituant : un atome de fluor, de chlore, de brome, un reste cyano, nitro, méthyle, éthyle, n- ou i-propyle, n-, i-, s- ou t-butyle, méthoxy, éthoxy, méthylthio, trifluorométhyle, trifluorométhoxy ou trifluorométhylthio, ou bien

$R^1$ et $R^2$ forment, avec l'atome d'azote auquel ils sont liés, un hétérocycle éventuellement substitué 1 à 3 fois, de façon identique ou différente et répondant à la formule :

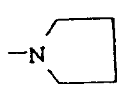 ; 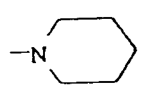 ; 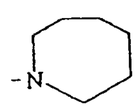 ; 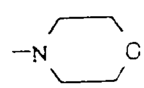 ;

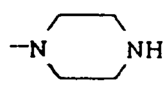 ; 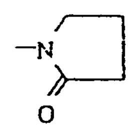 ; 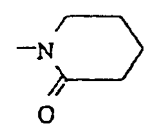 ; 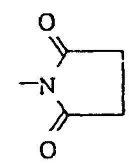 ;

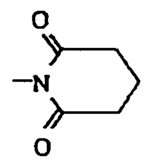 ; 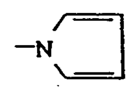 ou 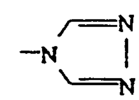

et l'on peut citer à chaque fois comme substituant : un reste méthyle, éthyle, n- ou i-propyle, chloro ou trifluorométhyle,

$R^3$ représente un groupe méthyle, éthyle, n- ou i-propyle, n-, i-, s- ou t-butyle, n- ou i-pentyle, n- ou i-hexyle, un groupe allyle, propargyle, méthoxyméthyle, un groupe halogénalkyle linéaire ou ramifié ayant 1 à 4 atomes de carbone et 1 à 9 atomes d'halogène identiques ou différents, un groupe cyclopentyle, cyclohexyle, cyclopropyle, cyclopropylméthyle, cyclohexylméthyle, cyclohexyléthyle ou un groupe benzyle ou phényle, chaque fois éventuellement substitué 1 à 3 fois de façon identique ou différente, et l'on peut citer comme substituant à chaque fois : un atome de fluor, de chlore, de brome, un reste cyano, nitro, méthyle, éthyle, n-ou i-propyle, n-, i-, s- ou t-butyle, méthoxy, éthoxy, méthylthio, trifluorométhyle, trifluorométhoxy ou trifluorométhylthio,

$R^4$ et $R^5$ représentent chacun, indépendamment l'un de l'autre, un atome d'hydrogène, un groupe méthyle, éthyle, n- ou i-propyle, n-, i-, s- ou t-butyle, un groupe, chaque fois linéaire ou ramifié, pentyle, hexyle, heptyle, octyle, nonyle, décyle ou dodécyle, un groupe allyle, n-ou i-butényle, n-ou i-pentényle, n- ou i-hexényle, propargyle, n- ou i-butynyle, n- ou i-pentényle, n- ou i-hexynyle, un groupe halogénoalkyle linéaire ou ramifié comportant 1 à 6 atomes de carbone et 1 à 9 atomes d'halogène identiques ou différents, un groupe halogéno-alcényle ou halogéno-alcynyle, chacun linéaire ou ramifié et comportant chacun 3 à 5 atomes de carbone et 1 à 3 atomes d'halogène, un groupe, chaque fois linéaire ou ramifié, cyanalkyle ayant 1 à 6 atomes de carbone dans la partie alkyle, hydroxyalkyle ayant 1 à 6 atomes de carbone et 1 à 3 groupes hydroxy, alcoxyalkyle, alcoxyminoalkyle, alcoxycarbonylalkyle ou alcoxycarbonylalcényle, alkylaminoalkyle ou dialkylaminoalkyle ayant à chaque fois jusqu'à 4 atomes de carbone dans les diverses parties alkyle ou alcényle, un groupe, chaque fois substitué 1 à 3 fois de façon identique ou différente, cyclopropyle, cyclopropylméthyle, cyclopropyléthyle, cyclobutyle, cyclopentyle, cyclohexyle, cycloheptyle, cyclooctyle, cyclohexylméthyle, cyclohexyléthyle, cyclohexényle, cyclohexénylméthyle ou cyclohexényléthyle, et l'on peut citer à chaque fois comme substituant : un atome de fluor, de chlore, de brome, un reste méthyle, éthyle, n- ou i-propyle, n-, i-, s- ou t-butyle, cyano, méthanediyle, éthanediyle, butanediyle ou butadiènediyle;

$R^4$ et $R^5$ peuvent représenter en outre un groupe (éventuellement substitué 1 à 3 fois, de façon identique ou différente, dans la partie hétérocyclyle), héterocyclylméthyle, hétérocyclylpropyle ou hétérocyclyléthyle, et l'on peut citer à chaque fois comme hétérocycle:

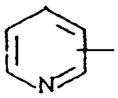 ; 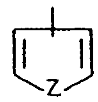 ; 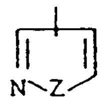 ; 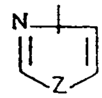 ;

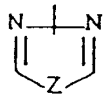 ; 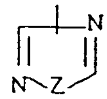 ; 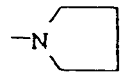 ; 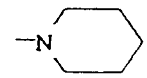 ;

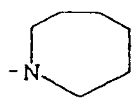 ; 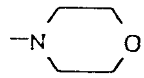 ou 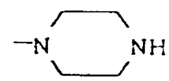 ,

où Z représente à chaque fois un atome d'oxygène ou de soufre, et l'on peut citer à chaque fois comme substituants :

un atome de fluor, de chlore, de brome, un reste cyano, nitro, méthyle, éthyle, n- ou i-propyle, n-, i-, s- ou t-butyle, méthoxy, éthoxy, méthylthio, trifluorométhyle, trifluorométhoxy ou trifluorométhylthio;

et, en outre, $R^4$ et $R^5$ peuvent représenter un groupe, chaque fois éventuellement substitué 1 à 3 fois de manière identique ou différente, éventuellement linéaire ou ramifié, benzyle, phényléthyle, phénylpropyle, phénylbutyle, phénylpentyle, phénylhexyle, phénylheptyle, phénylcyanométhyle, phénylcyanométhyle, phénylcyanoéthyle, phénylcyanopropyle, benzoyle, phényle ou naphtyle, et l'on peut citer à chaque fois comme substituant fixé sur le noyau phényle : un atome de fluor, de chlore, de brome, un reste hydroxyle, cyano, nitro, méthyle, éthyle, n- ou i-propyle, n-, i-, s- ou t-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy, ou bien

$R^4$ et $R^5$ forment, avec l'atome d'azote auquel ils sont liés, un hétérocycle éventuellement substitué 1 à 3 fois de façon identique ou différente, et qui répond à la formule

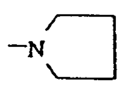 ; 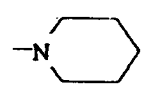 ; 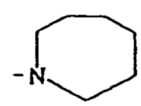 ; 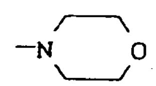 ;

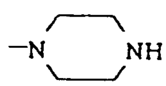 ; 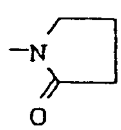 ; 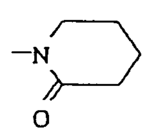 ; 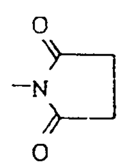 ;

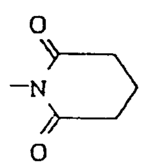 ; 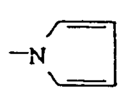 ou 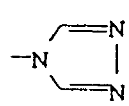

où l'on peut citer à chaque fois comme substituants : un reste méthyle, éthyle, n- ou i-propyle, chloro ou trifluorométhyle, et

X représente un atome d'oxygène ou de soufre, à l'exclusion des composés dans

lesquels

$R^1$ et $R^2$ forment, avec l'atome d'azote auquel ils sont liés, un hétérocycle éventuellement substitué,

X représente un atome de soufre,

$R^3$ représente un groupe aryle ayant 6 à 10 atomes de carbone,

$R^4$ et $R^5$ représentent chacun, indépendamment l'un de l'autre, un atome d'hydrogène, un groupe alkyle à chaque fois linéaire ou ramifié et comportant 1 à 18 atomes de carbone, et, en outre, $R^4$ et $R^5$ peuvent représenter un groupe aryle ou aralkyle, éventuellement substitué une ou plusieurs fois de façon identique ou différente et comportant à chaque fois 6 à 10 atomes de carbone dans la partie aryle et éventuellement 1 à 8 atomes de carbone dans la partie alkyle linéaire ou ramifiée.

**3.** Triazoles substitués de formule (I) selon la revendication 1, dans laquelle

$R^1$ représente un atome d'hydrogène, un groupe méthyle ou éthyle,

$R^2$ représente un groupe méthyle ou éthyle,

$R^3$ représente un groupe méthyle ou éthyle,

$R^4$ représente un atome d'hydrogène ou un groupe méthyle,

$R^5$ représente un atome d'hydrogène, un groupe méthyle, éthyle, n-ou i-propyle, n-, i-, s- ou t-butyle, un groupe, à chaque fois linéaire ou ramifié, pentyle, hexyle, heptyle, octyle, nonyle, décyle ou dodécyle, allyle, n- ou i-buténыle, n- ou i-penténыle, n- ou i-hexényle, propargyle, n- ou i-butynyle, n- ou i-pentynyle, n- ou i-hexynyle, un groupe halogéno-alkyle linéaire ou ramifié comportant 1 à 6 atomes de carbone et 1 à 9 atomes d'halogène identiques ou différents, un groupe halogéno-alcényle ou halogéno-alcynyle, à chaque fois linéaire ou ramifié et comportant chacun 3 à 5 atomes de carbone et 1 à 3 atomes d'halogène, un groupe, chaque fois linéaire ou ramifié, cyanalkyle ayant 1 à 6 atomes de carbone dans la partie alkyle, hydroxyalkyle ayant 1 à 6 atomes de carbone et 1 à 3 groupes hydroxyle, alcoxyalkyle, alcoxyiminoalkyle, alcoxycarbonylalkyle ou alcoxycarbonylalcényle, alkylaminoalkyle ou dialkylaminoalkyle ayant chacun jusqu'à 4 atomes de carbone dans les diverses parties alkyle ou alcényle ou un groupe (éventuellement substitué 1 à 3 fois, de façon identique ou différente, par un atome de fluor, de chlore, de brome, par un reste méthyle, éthyle, n- ou i-propyle, n-, i-, s-ou t-butyle, cyano, méthanediyle, éthanediyle, butanediyle ou butadiènediyle) cyclopropyle, cyclopropylméthyle, cyclopropyléthyle, cyclobutyle, cyclopentyle, cyclohexyle, cycloheptyle, cyclooctyle, cyclohexylméthyle, cyclohexyléthyle, cyclohexényle, cyclohexénylméthyle ou cyclohexényléthyle ; $R^5$ représente en outre un groupe (éventuellement substitué 1 à 3 fois dans la partie hétérocyclyle, de façon identique ou différente, par un atome de fluor, de chlore, de brome, un reste cyano, nitro, méthyle, éthyle, n-ou i-propyle, n-, i-, s- ou t-butyle, méthoxy, éthoxy, méthylthio, trifluorométhyle, trifluorométhoxy ou trifluorométhylthio), hétérocyclylméthyle, hétérocyclylpropyle ou hétérocyclyléthyle, et l'on peut citer comme hétérocycle :

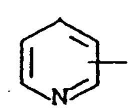 ; 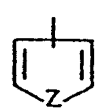 ; 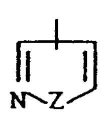 ; 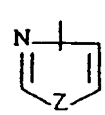 ;

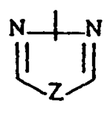 ; 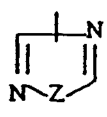 ; 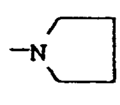 ; 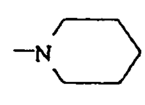 ;

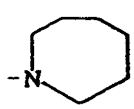 ; 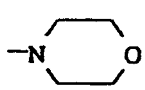 ou 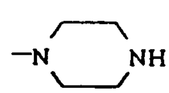 ,

où Z représente à chaque fois un atome d'oxygène ou de soufre, et, en outre, $R^5$ peut représenter un groupe (chaque fois éventuellement substitué dans la partie phényle, 1 à 3 fois de façon identique ou différente par un atome de fluor, de chlore, de brome, par un groupe hydroxy, cyano, nitro, méthyle, éthyle, n- ou i-propyle, n-, i-, s-ou t-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy) éventuellement linéaire ou ramifié, benzyle, phényléthyle, phénylpropyle, phénylbutyle, phénylpentyle, phénylhexyle, phénylheptyle, phénylcyanométhyle, phénylcyanéthyle, phénylcyanopropyle, benzoyle, phényle ou naphtyle, et,

X représente un atome d'oxygène, à l'exclusion des composés dans lesquels

$R^1$ et $R^2$, pris avec l'atome d'azote auquel ils sont reliés, forment un hétérocycle éventuellement substitué;

X représente un atome de soufre;

$R^3$ représente un groupe aryle ayant 6 à 10 atomes de carbone,

$R^4$ et $R^5$ représentent chacun, indépendamment l'un de l'autre, un atome d'hydrogène, un groupe alkyle linéaire ou ramifié ayant 1 à 18 atomes de carbone et, en outre, un groupe aryle ou aralkyle, éventuellement substitué une ou plusieurs fois de façon identique ou différente et comportant chacun 6 à 10 atomes de carbone dans la partie aryle et éventuellement 1 à 8 atomes de carbone dans la partie alkyle linéaire ou ramifiée.

**4.** Procédé pour produire des triazoles substitués de formule (I)

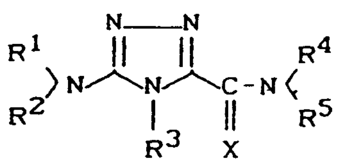

(I)

dans laquelle

$R^1$, $R^2$, $R^3$, $R^4$, $R^5$ et X ont les définitions citées à la revendication 1, à l'exclusion des composés dans lesquels

$R^1$ et $R^2$ forment, avec l'atome d'azote auquel ils sont reliés un hétérocycle éventuellement substitué,

X représente un atome de soufre,

$R^3$ représente un groupe aryle ayant 6 à 10 atomes de carbone,

$R^4$ et $R^5$ représentent chacun, indépendamment l'un de l'autre, un atome d'hydrogène, un groupe alkyle linéaire ou ramifié ayant 1 à 18 atomes de carbone, et, en outre, un groupe aryle ou aralkyle éventuellement substitué une ou plusieurs fois de façon identique ou différente et comportant chacun 6 à 10 atomes de carbone dans la partie aryle et éventuellement 1 à 8 atomes de carbone dans la partie alkyle linéaire ou ramifiée,

procédé caractérisé en ce que

(a) on fait réagir en présence d'un diluant et éventuellement en présence d'un adjuvant de réaction, des aminoguanidines de formule (II)

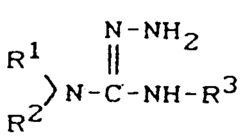

(II)

dans laquelle

$R^1$, $R^2$ et $R^3$ ont le sens indiqué ci-dessus,

ou leurs sels d'addition d'acide, avec des (thio)amides d'esters oxaliques de formule (III)

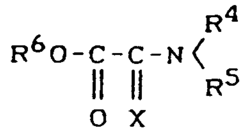

(III)

dans laquelle

$R^6$ représente un groupe alkyle, et

$R^4$, $R^5$ et X ont le sens indiqué ci-dessus,

ou bien

(b) on fait réagir, éventuellement en présence d'un diluant, des esters d'acide triazolyl(thio)-carboxyliques substitués, de formule (IV)

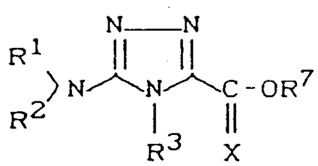

(IV)

dans laquelle

$R^7$ représente un groupe alkyle, et

$R^1$, $R^2$, $R^3$ et X ont le sens indiqué ci-dessus,

avec des amines de formule (V)

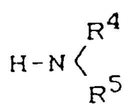

(V)

dans laquelle

$R^4$ et $R^5$ ont le sens indiqué ci-dessus.

**5.** Produit herbicide, caractérisé par une teneur en au moins un triazole substitué de formule (I) selon les revendications 1 à 4.

**6.** Procédé pour combattre des mauvaises herbes, caractérisé en ce qu'on fait agir sur les mauvaises herbes et/ou sur leur espace vital ou biotope des triazoles substitués de formule (I) selon les revendications 1 à 4.

**7.** Utilisation de triazoles substitués, répondant à la formule (I) selon les revendications 1 à 4, pour lutter contre les mauvaises herbes.

**8.** Procédé pour préparer des produits herbicides, caractérisé en ce qu'on mélange des triazoles substitués de formule (I) selon les revendications 1 à 4 avec des agents d'allongement et/ou avec des substances tensioactives.

**9.** Les composés de formule

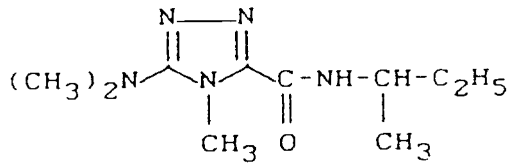

et